(19) Europäisches Patentamt
European Patent Office
Office européen des brevets

(11) Numéro de publication: **0 234 151**
**B1**

(12) # FASCICULE DE BREVET EUROPEEN

(45) Date de publication du fascicule du brevet: **12.12.90**

(51) Int. Cl.[5]: **C07K 15/14**, A61K 37/02

(21) Numéro de dépôt: **86402812.1**

(22) Date de dépôt: **16.12.86**

(54) **Glycoprotéines modifiées par oxydation et formation de base de Schiff, inhibant les ribosomes, procédé d'obtention et immunotoxines comprenant une telle glycoprotéine.**

(30) Priorité: **20.12.85 FR 8518981**
**12.08.86 FR 8611643**

(43) Date de publication de la demande:
**02.09.87 Bulletin 87/36**

(45) Mention de la délivrance du brevet:
**12.12.90 Bulletin 90/50**

(84) Etats contractants désignés:
**AT BE CH DE ES FR GB GR IT LI LU NL SE**

(56) Documents cités:
**EP-A- 0 074 279**
**EP-A- 0 129 434**
**GB-A- 1 446 536**

**CHEMICAL ABSTRACTS, vol. 88, no. 25, 19 juin 1978, page 213, résumé no. 184277s, Columbus, Ohio, US; K. SANDVIG et al.: "Chemical modifications of the toxic lectins abrin and ricin", & EUR. J. BIOCHEM. 1978, 84(2), 323-31**

(73) Titulaire: **SANOFI, 40, Avenue George V, F-75008 Paris(FR)**

(72) Inventeur: **Casellas, Pierre, La Colombe no. 9 Rue Aiguelongue, F-34100 Montpellier(FR)**
Inventeur: **Bourrie, Bernard, Résidence Du plan des 4 Seigneurs 2 batiment B, 30, rue des Brusses F-34100 Montpellier(FR)**
Inventeur: **Canat, Xavier, 4, rue des Carignans, F-34680 Saint Georges D'Orques(FR)**

(74) Mandataire: **Gillard, Marie-Louise et al, Cabinet Beau de Loménie 55, Rue d'Amsterdam, F-75008 Paris(FR)**

**EP 0 234 151 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne de nouvelles molécules médicamenteuses comportant au moins un anticorps lié de façon covalente à un constituant de nature polypeptidique, inhibiteur de la synthèse protéique et issu d'une glycoprotéine (ou d'un glycopeptide) dont les motifs polysaccharidiques ont été modifiés.

Dans le brevet US 4 340 535 et dans les demandes de brevet français publiées sous les n° 2 504 010 et 2.516 794 est décrite la préparation de produits anti-cancéreux dits conjugués obtenus par couplage, par liaison covalente, de la chaîne A de ricine avec des anticorps ou fragments d'anticorps dirigés contre des antigènes portés par la cellule à détruire. Les produits de ce type ont été désignés et sont désignés dans la présente demande sous le nom générique d'Immunotoxines.

On connaît aussi des conjugués analogues aux immunotoxines à chaîne A de ricine précédemment décrites, ayant aussi vocation de médicaments anti-cancéreux et résultant du couplage par liaison covalente d'anticorps ou fragments d'anticorps à d'autres glycoprotéines inactivant les ribosomes tels que notamment la gélonine extraite de Gelonium multiflorum (Eur. J. Biochem, 1981, 116, 447-454 ; Cancer Res. 1984, 44, 129-133) ou l'inhibiteur extrait de Momordica Charantia (MOM) (brevet US 4 368 149).

Cas glycoprotéines inactivant les ribosomes (abrégé GPIR) qui ont des propriétés semblables à celles de la chaîne A de ricine sont des substances de poids moléculaire d'un ordre de grandeur de 20.000 et 30.000 (Cancer Survey 1982, 1, 489-520).

Le terme "glycoprotéine inactivant les ribosomes" (GPIR), tel qu'utilisé dans la présente description ainsi que dans les revendications, désigne toute substance portant des motifs saccharidiques appartenant à la classe des macromolécules inactivant les ribsomes et, par conséquent, inhibitant la synthèse protéique de cellules eucaryotes, ainsi que tout fragment de ladite substance possédant la même propriété inactivante, ladite glycoprotéine inactivant les ribosomes pouvant être d'origine naturelle ou biosynthétique provenant d'une cellule dont le patrimoine génétique a été modifié dans ce but.

On sait également que l'activité cytotoxique de certaines immunotoxines peut être potentialisée par diverses substances adjuvantes telles que les sels d'ammonium, diverses amines ou certains ionophores carboxyliques tels que la monensine ou la nigéricine.

Toutefois, les effets thérapeutiques des immunotoxines, activées ou non, ne peuvent se manifester pleinement que dans la mesure où l'immunotoxine peut, par sa partie anticorps, se localiser in vivo, sous forme active, sur la cible cellulaire à déctruire (condition sine qua non à toute expression d'activité des immunotoxines). La capacité de l'immunotoxine à se localiser sur la cible dépend en tout premier lieu de l'aptitude de l'immunotoxine à demeurer dans la circulation sanguine et les fluides extra-cellulaires, sous forme active, pendant des temps suffisants pour qu'elle atteigne sa cible cellulaire et à des concentrations suffisamment fortes pour que le taux d'occupation des sites antigéniques correspondants soit élévé.

De nombreuses études ont permis d'établir la cinétique d'élimination plasmatique des immunotoxines après injection intraveineuse dans différents modèles animaux. Il est apparu qu'après l'injection le taux plasmatique d'immunotoxine biologiquement active décroît très rapidement et de façon très importante. Ainsi, typiquement, chez le lapin, dans un modèle utilisant une immunotoxine construite en couplant, à l'aide d'un bras à pont disulfure, la chaîne A de ricine à un anticorps monoclonal dirigé contre l'antigène T65 des lymphocytes T humains (anticorps T101), il apparaît que 97 % de l'immunotoxine présente dans le sang circulant au temps 0 de l'injection disparaissent en 30 min et 99,9 % en 17 h. Cette rapide disparition de l'immunotoxine est bien évidemment préjudiciable à l'expression de sa capacité cytotoxique complète, en empêchant que l'immunotoxine ne sature de façon durable une proportion élevée des antigènes-cibles portés par les cellules à détruire. En outre, la comparaison des cinétiques d'élimination plasmatique des immunotoxines avec celles des anticorps non conjugués correspondants montre qu'à l'inverse les anticorps -comme cela est bien connu- se maintiennent dans le plasma à un haut niveau pendant des temps relativement longs. Or il existe toujours, même dans les préparations d'immunotoxines les plus purifiées, un certain taux résiduel d'anticorps non conjugués. Par le jeu des vitesses différentielles d'élimination des immunotoxines et des anticorps, progressivement les anticorps non conjugués, initialement très minotaires, deviennent majoritaires au bout de quelques heures et donc, progressivement, ces anticorps deviennent par compétition de puissants antagonistes pour la fixation des immunotoxines sur leurs cibles.

Il apparaît donc clairement l'intérêt d'accroître la persistance plasmatique des immunotoxines, sous forme active afin d'augmenter à la fois la durée et le taux d'occupation des antigènes-cibles et par conséquent d'améliorer les effets thérapeutiques des immunotoxines.

Par ailleurs, des expériences de localisation in vivo de l'immonotoxine à chaîne A de ricine, radiomarqués puis injectée chez des animaux sans cible spécifique, ont montré que, dans les premières minutes après l'injection, le conjugé se localise préférentiellement dans le foie. Il en est de même pour la chaîne A de ricine qui présente le même devenir lorsqu'elle est injectée sous forme non couplée. Cela suggère fortement que l'immunotoxine se fixe dans le foie par l'intermédiaire de la sous-inité cytotoxique qu'elle contient.

Il est connu que la chaîne A de ricine est une glycoprotéine dont les groupements polyosidiques comprennent notamment des résidus de mannose et de N-acétylglucosamine, certains résidus de mannose

étant en positions terminales (agri. Biol. Chem., 1978, 42, 501). Il a été également établi l'existence au niveau du foie de récepteurs capables de reconnaître des glycoprotéines ayant de tels résidus de mannose terminaux. Il a été aussi montré que les glycoprotéines reconnues par ces recepteurs -présents essentiellement sur les cellules de Kupffer- sont rapidement éliminées de la circulation sanguine par fixation sur ces cellules qui les métabolisent. Ceci est bien documenté notamment dans le cas des bêta glucuronidase, ainsi que dans le cas de la ribonucléase B (Arch. Biochem. Biophys., 1978, 188, 418 ; Advances in Enzymology, Meister A. Ed., New York, 1974 ; Pediat. Res., 1977, 11, 816).

De l'ensemble de ces données, il apparaît que la rapide élimination des immunotoxines à chaîne A de ricine peut s'expliquer par la reconnaissance des résidus mannose de la chaîne A de ricine par les cellules hépatiques et en particulier les cellules de Kupffer.

Les études de cinétique d'élimination plasmatique d'autres GPIR, par exemple la gélonine ou le MOM (substance extraite de Monocardia Charantia), après injection intraveineuse chez l'animal, ont montré que, comme dans le cas de la chaîne A de ricine, le taux plasmatique de GPIR décroît très rapidement et de façon très importante après l'injection. Ainsi typiquement chez le lapin après injection de gélonine purifiée selon la méthode décrité (J. Biol. Chem., 1980, 255, 6947-6953) il apparaît que 93 % de la gélonine présente dans le sang circulant au temps 0 de l'injection disparaissent en 1 h et 99,99 % en 24 h.

Il est connu que par oxydation par les ions periodate des structures osidiques, y compris celles qui sont contenues dans les glycoprotéines, on provoque la rupture de la chaîne carbonée chaque fois que deux atomes de carbone adjacents portent des hydroxyles primaires ou secondaires. Si les deux hydroxyles contigus sont secondaires, comme cela est généralement le cas dans les oses cycliques présents dans les GPIR, l'oxydation conduit à deux fonctions aldéhyde sur les carbones entre lesquels la rupture a eu lieu.

D'autre part, on sait que les fonctions aldéhyde sont très actives vis-à-vis des groupements amines primaires par formation d'imines également connues sous le nom de bases de Schiff. Ainsi, les groupes aldéhyde formés lors de la réaction d'oxydation peuvent réagir avec des amines primaires portées par la chaîne peptidique de la glycoprotéine, et former des liaisons covalentes intra- et/ou intermoléculaires indésirables, conduisant à l'instabilité du produit d'oxydation et fréquemment à la formation de polymères insolubles.

On a maintenant trouvé, de façon absolument inattendue que lorsqu'on modifie les motifs glucidiques d'une GPIR par le procédé original décrit ci-après, on obtient une nouvelle molécule de GPIR ayant la double propriété de conserver ses activités biologiques et d'être éliminée très lentement de la circulation sanguine après injection chez les animaux supérieurs ou l'homme. Cette nouvelle GPIR modifiée qui conserve la propriété d'inactiver les ribosomes et qui a acquis, du fait de la modification, une longue durée d'action in vivo est désignée dans la présente demande par le symbole GPIR-La.

Ce procédé original consiste à modifier les motifs osidiques de la GPIR par réaction avec des ions periodate en présence d'un excès d'un réactif auxiliaire comportant un groupement amine primaire et qui, par le reste de la structure, n'est pas susceptible de réagir avec les ions periodate dans les conditions opératoires utilisées. De la sorte, les groupements aldéhyde engendrés par l'oxydation periodique sont bloqués au fur et à mesure de leur apparition par formation de base de Schiff avec le réactif aminé exogène en excès, évitant ainsi les réactions indésirables avec d'autres groupes aminés de la GPIR et permettant d'obtenir un produit stable et bien soluble.

On a également trouvé que, lorsque cette nouvelle molécule de GPIR à longue durée d'action est couplée à des anticorps ou fragments d'anticorps, les conjugués obtenus conservent les propriétés biologiques connues des immunotoxines et présentent une cinétique d'élimination plasmatique lente.

La présente invention a donc pour objet, à titre de produit nouveau, une GPIR modifée dans sa structure, dont les motifs glucidiques ont été modifiés par l'action des ions periodate en présence d'un excès de réactif auxiliaire présentant un groupe amine primaire et capable de bloquer les groupements aldéhyde engendrés par l'oxydation periodique, au fur et à mesure de leur apparition. La présente invention a aussi pour objet le procédé d'obtention de la GPIR modifiée telle que caractérisée ci-dessus.

La présente invention a également pour objet des produits appartenant à la classe des immunotoxines, obtenus par couplage covalent entre, d'une part, un anticorps ou fragment d'anticorps utilisé sous forme naturelle ou correctement modifié et, d'autre part, une molécule de GPIR dont les motifs glucidiques ont été modifiés par le procédé original ci-dessus.

On précise ci-après la signification des différents produits utilisés dans la mise en oeuvre de l'invention :

Le terme "periodate" désigne l'ion $IO_4$-présent dans les solutions aqueuses des sels de l'acide periodique et notamment les sols dérivant des métaux alcalins. Ces sels sont également mentionnés dans la littérature sous le nom de métaperiodates.

Le terme "réactif auxiliaire présentant un groupe amine primaire" désigne toute molécule organique porteuse d'un unique groupe amine primaire qui n'est pas susceptible de réagir avec les ions periodate dans les conditions opératoires utilisées. en particulier ce réactif peut être toute alkylamine, non réactive avec le periodate et soluble dans l'eau, à la concentration nécessaire, sous forme de base ou de sel, à pH compris entre 5 et 7 et à température comprise entre 0 et 15°C. Ce réactif peut être aussi tout acide $\alpha$ ou $\omega$ aminé répondant aux conditions ci-dessus, qu'il soit optiquement actif ou racémique, et notamment l'un des acides aminés aliphatiques suivants : glycine, alanine, valine, leucine, isoleucine, arginine,

acide aspartique, acide glutamique. Ce réactif peut être aussi tout peptide répondant aux conditions ci-dessus fixées.

Le terme "anticorps" représente une protéine choisie parmi tout anticorps ou fragment d'anticorps, ou toute immunoglobuline ou fragment d'immunoglobuline ou toute molécule, dérivant des précédentes par modification artificielle de l'un quelconque de leurs groupements fonctionnels, y compris des structures osidiques qu'elles portent, sous réserve que la protéine ainsi choisie reste capable de reconnaître sélectivement un antigène donné à la surface des cellules porteuses de cet antigène et notamment des cellules cibles. L'anticorps de départ peut être d'orgine naturelle ou biosynthétique provenant d'une cellule dont le patrimoine génétique a été modifié dans ce but.

La préparation d'anticorps monoclonaux dirigés notamment contre des cellules cibles humaines définies a été largement mentionnée dans la littérature scientifique et beaucoup de ces anticorps sont maintenant disponsibles dans le commerce.

Le symbole P représente une protéine choisie parmi tout anticorps ou fragment d'anticorps, ou toute immunoglobuline ou fragment d'immunoglobuline ou toute molécule dérivant des précédentes par modification artificielle de l'un quelconque de leurs groupements fonctionnels, y compris des structures glucidiques qu'elles portent, sous réserve que la protéine ainsi choisie reste capable de reconnaître sélectivement un antigène donné à la surface des cellules porteuses de cet antigène et notamment des cellules cancéreuses. La protéine P de départ peut être d'origine naturelle ou biosynthétique provenant d'une cellule dont le patrimoine génétique a été modifié dans ce but.

Le terme "GPIR" représente une glycoprotéine ou un de ses fragments. A condition que de tels fragments retiennent tout ou partie de la propriété d'inactivation des ribosomes qui caractérise la GPIR dont ils sont issus, ils peuvent être utilisés comme produit de départ ; on préfère toutefois la GPIR native.

Le terme "GPIR-La" représente la GPIR modifiée selon l'invention, c'est à dire une molécule ayant la propriété d'inactiver les ribosomes comme la GPIR mais ayant une durée d'action in vivo supérieure à celle de la GPIR et qui résulte du traitement en milieu aqueux de la GPIR par un agent oxydant, comme le periodate, en présence d'un excès d'amine primaire pour transformer les groupes aldéhyde formés en groupes imine (ou base de Schiff).

On opère en général à une température comprise entre 0 et 15°C, dans une solution aqueuse de pH compris entre 5 et 7, et de préférence à l'abri de la lumière ; dans ces conditions la réaction dure de 0,2 à 24 heures.

Dans l' immunotoxine, la partie GPIR-La est également indiquée comme "sous-unité cytotoxique".

Le symbole A-La représente une glycoprotéine inactivant les ribosomes et à longue durée d'action, obtenue par traitement de la chaîne A de ricine, dont au moins un des groupes thiol de ses cystéines 171 et 257 est éventuellement protégé, avec une solution aqueuse d'un periodate alcalin en présence d'une amine primaire en excès pendant une période de temps de 0,2 à 24 h à la température de 0 à 15° C et à l'abri de la lumière et par déprotection éventuelle dudit groupe thiol.

Le symbole P' représente un radical dérivé de la protéine P ci-dessus telle quelle ou chimiquement convenablement modifiée, privée d'une ou plusieurs fonctions propres et dont d'autres groupes fonctionnels sont éventuellement bloqués.

Le symbole GPIR-La' représente un radical dérivé de la protéine GPIR-La ci-dessus telle quelle ou chimiquement convenablement modifiée, privée d'une ou plusieurs fonctions propres et dont d'autres groupes fonctionnels sont éventuellement bloqués.

Le symbole A-La' représente un radical dérivé de la protéine A-La privé d'au moins une des fonctions thiol de ses cystéines 171 et 257.

Le symbole $P_1$ représente une des protéines GPIR-La et P telles que définies ci-dessus qui porte des fonctions thiol libres, attachées à ladite protéine directement ou par l'intermédiaire d'une structure d'espacement.

Le symbole $P_2$, différent de $P_1$, représente une des protéines GPIR-La et P telles que définies ci-dessus, qui porte un ou plusieurs groupements fonctionnels capables de réagir avec des thiols libres.

Le symbole $P_1$' représente le radical de la protéine $P_1$ lié aux fonctions propres de la protéine $P_1$ notamment les fonctions SH (de la cystéine), $NH_2$ (terminal de la protéine ou dans la position epsilon des lysines), OH (des tyrosines), COOH (des acides aspartiques et glutamique) ou, seulement dans le cas où $P_1$ est un anticorps ou fragment d'anticorps, le radical de la protéine $P_1$ provenant de l'ouverture des structures glucidiques par action de l'acide periodique selon les méthodes connues.

Le symbole $P_2$' représente le radical de la protéine $P_2$ lié aux groupes fonctionnels caractéristiques $NH_2$ (terminal de la protéine ou dans la position epsilon des lysines), OH (des tyrosines), COOH (des acides aspartiques et glutamiques).

Par exemple, $P_1$'-SH représente la protéine $P_1$ (qui peut être indifféremment l'anticorps ou fragment d'anticorps P ou la GPIR-La) dans laquelle les groupes SH des cystéines sont libres et les autres groupes fonctionnels sont éventuellement bloqués.

De la même façon, $P_1$'-CO- représente la protéine $P_1$, dans laquelle son groupe carboxylique terminal ou les groupes carboxyliques de ses acides glutamiques et aspartiques sont couplés avec un groupement qui apporte un groupe SH introduit artificiellement.

Encore $P_2$'-NH- représente la protéine $P_2$ (qui peut être indifféremment l'anticorps ou fragment d'anticorps P ou la GPIR-La) dans laquelle son groupe amino terminal ou les groupes amino de ses lysines sont attachés à une groupement susceptible de couplage avec le thiol de la protéine $P_1$.

Le terme "structure" d'espacement inerte" tel qu'utilisé ici pour E et E' désigne un radical organique bivalent inerte vis-à-vis des réactifs utilisés dans le procédé, tel qu'un groupe alkylène à chaîne droite ou ramifiée contenant de 1 à 15 atomes de carbone, qui peut contenir une ou plusieurs doubles liaisons ou qui peut être interrompu par des atomes d'oxygène ou qui peut être substitué par un ou plusieurs groupes fonctionnels inertes, tels que des groupes méthoxy, des groupes carboxyles libres ou estérifiés, des groupes dialkylamino, des groupes carbamates. Le même terme désigne également un groupe arylène contenant de 6 à 15 atomes de carbone qui peut être substitué par un ou plusieurs groupes fonctionnels inertes comme indiqué ci-dessus pour le groupe alkylène.

L'expression "groupe fonctionnel capable de se lier d'une façon covalente" telle qu'utilisée ici pour Y et Y' désigne tous les groupes susceptibles de réagir avec les fonctions propres des protéines $P_1$ et $P_2$ pour donner une liaison covalente. Ainsi, les groupes -CO- et -C=NH)- sont des groupes fonctionnels, convenables susceptibles de se lier aux amines libres, les thiols et les hydroxyles phénoliques des protéines. De même, le groupe -NH- est un groupe fonctionnel convenable, susceptible de se lier aux groupes carboxyliques libres des protéines. Le groupe =N- est une groupe fonctionnel convenable, susceptible de se lier aux deux atomes de carbone des structures glucidiques des protéines $P_1$ et $P_2$ après oxydation avec les ions periodate, mais seulement dans le cas où $P_1$ et $P_2$ sont un anticorps ou un fragment d'anticorps.

L'expression "fonction propre des protéines", telle qu'utilisée ici pour Z et Z', désigne les radicaux provenant des groupes caractéristiques des acides aminés formant les protéines $P_1$ et $P_2$, telles que l'atome d'oxygène provenant des hydroxyles des acides aminés tyrosine et éventuellement sérine, le groupe carbonyle provenant du carboxyle terminal ou des carboxyles libres des acides aspartique et glutamique, le groupe -NH- provenant de l'amine terminale des protéines par exemple de la lysine, l'atome de soufre provenant du thiol de la cystéine. La même expression désigne également le groupe provenant de la structure dialdéhydique obtenue après oxydation d'une des structures glucidiques des protéines $P_1$ et $P_2$ par traitement avec les ions periodate, mais seulement dans le cas où les $P_1$ et $P_2$ sont un anticorps ou fragment d'anticorps.

Le terme "radical activateur", tel qu'utilisé ici pour X, désigne un groupement lié à un pont -S-S- capable de réagir avec un thiol libre pour former un disulfure avec libération de X-SH. Des radicaux activateurs convenables sont les groupes pyridyl-2 et pyridyl-4, non substitués ou substitués par un ou des halogènes ou des radicaux alkyle, carboxyle, alcoxycarbonyle ; le groupe phényle non substitué ou, de préférence, susbtitué par un ou des halogènes ou des groupes nitro, alcoxy, carboxyle ou alcoxycarbonyle ; ou un groupe alcoxycarbonyle tel que méthoxycarbonyle.

Les termes "alkyle" et "alcoxy" désignent des groupes contenant jusqu'à 5 atomes de carbone.

Le terme "alkylène" désigne des groupements aliphatiques saturés, à chaîne droite ou ramifiée, contenant jusqu'à 10 atomes de carbone pouvant être substitués par un ou plusieurs groupes fonctionnels inertes tels que les groupes alcoxycarbonyle.

Des glycoprotéines inactivant les ribosomes utilisées comme produits de départ préférentiels pour l'oxydation par les ions periodate et la réduction selon l'invention sont toutes les GPIR, comme la chaîne A de ricine, qui sont par elles-mêmes très faiblement cytotoxiques car elles ne peuvent se fixer sur les cellules, mais qui, par contre, après couplage à un anticorps reconnaissant des cellules particulières, deviennent hautement cytotoxiques pour ces cellules une fois que l'anticorps a reconnu sa cible.

Les composés de départ représentatifs sont la chaîne A de ricine, la gélonine et la substance extraite de Momordica Charantia (MOM), telles qu'elles sont obtenues par la voie extractive.

D'autres GPIR utiles comme produits de départ pour l'oxydation par les ions periodate sont les suivants :

Dianthin 30 : de Dianthus caryophyllus
Dianthin 32 : de Dianthus caryophyllus
Agrostin A : de Agrostemma gitnago
Agrostin B : de Agrostemma gitnago
Agrostin C : de Agrostemma gitnago
HCI : de Hura crepitans
Asparagus officinalis : de Asparagus officinalis.

Les mêmes substances, produites par la voie biosynthétique par des cellules appropriées dont le patrimoine génétique a été modifié dans ce but, sont des composés également convenables.

Des fragments des GPIR ci-dessus, à condition que de tels fragments retiennent tout ou partie de la propriété d'inactivation des ribosomes qui caractérise la GPIR dont ils sont issus, peuvent également être utilisés comme produit de départ.

La chaîne A de ricine native, dont au moins l'un des groupements thiol est protégé, est un composé de départ préférentiel.

L'obtention de la chaîne A de la ricine pure est décrite dans le brevet US 4 340 535. La gélonine et le MOM sont également décrits.

La protection des groupes thiol des GPIR de départ est souhaitable lorsque lesdits groupes thiol sont ceux qui serent utilisés pour le couplage avec l'anticorps. Si pour le couplage on utilise d'autres groupes

fonctionnels, par exemple l'hydroxyle phénolique des tyrosines ou des groupes aminés ou des groupes carboxyliques de la GPIR, la protection n'est pas effectuée.

Le blocage est effectué par réaction avec un agent susceptible de substituer les fonctions SH à l'aide d'un radical qui peut être ensuite éliminé par réduction ou échange thiol/disulfure, par exemple l'acide dinitro-2,2' dithio-5,5' dibenzoïque (DTNB) ou bien l'acide (pyridyl=2 disulfanyl)-3 propionique. En l'absence d'un tel traitement, les thiols libres peuvent disparaître pendant la réaction d'oxydation et dans ce cas ne peuvent être totalement régénérés. L'excès de l'agent bloquant est éliminé par dialyse ou tout autre traitement approprié.

La GPIR dont les thiols sont éventuellement bloqués est alors soumise à la réaction d'oxydation par les ions periodate et à la formation simultanée de base de Schiff avec une amine primaire.

La réaction d'oxydation periodique et la réaction de blocage par formation de base de Schiff peuvent être effectuées à un pH modérement acide, compris entre 5 et 7, de préférence entre 6 et 6,5. La GPIR est mélangée à l'amine primaire avant l'addition du periodate. Le periodate est utilisé en excès ; plus particulièrement, la concentration en periodate alcalin est toujours supérieure à la concentration des diols vicinaux susceptibles d'être oxydés : des concentrations de 10 à 50 mM en periodate de sodium pour des concentrations de 1 à 10 mg/ml de sous-unité cytotoxique sont convenables. L'amine primaire (telle que la L-leucine, la L-alanine ou la L-arginine ou l'acide L-glutamique) est également utilisée à une concentration supérieure à la concentration des diols vicinaux susceptibles d'être oxydés : des concentrations de 50 à 500 mM en amine primaire pour des concentrations de 1 à 10 mg/ml de sous-unité cytotoxique sont convenables. La durée du traitement, réalisé à une température comprise entre 0 et 15°C et de préférence entre 1 et 5°C et à l'obscurité, est comprise entre 0,2 et 24 heures.

Lorsque la réaction est terminée, l'excès de periodate est éliminé par addition d'un réactif qui le consomme, par exemple un excès d'éthylène-glycol et les sous-produits sont éliminés par dialyse. Le produit obtenu à la fin de la réaction est isolé selon les techniques classiques.

Lorsque les groupes thiol du produit de départ ont été bloqués, le déblocage est effectué selon les méthodes connues, par exemple par action d'un agent réducteur apte à libérer la fonction thiol préalablement bloquée, tel que le mercapto-2 éthanol, ce qui conduit à l'obtention de la nouvelle glycoprotéine inactivant les ribosomes et à longue durée d'action prête à être utilisée pour le couplage avec un anticorps pour obtenir une immunotoxine.

Dans le cas de la chaîne A de la ricine, la nouvelle molécule de la chaîne A de ricine ainsi obtenue (A-La) possède les propriétés principales suivantes :

- poids moléculaire non significativement différent de celui de la chaîne A native. Par électrophorèse en gradient de polyacrylamide du produit final, on constate que le procédé de modification ne fournit des polymères de la protéine qu'en quantité nulle ou très faible et ne fournit aucun produit de dégradation ;
- un taux de groupements thiol libres supérieur à 0,7 par mole ;
- une immunoréactivité envers des anticorps de lapin anti-chaîne A de ricine que l'on ne peut distinguer de celle de la chaîne A native ;
- une activité inhibitrice de la synthèse protéique dans un modèle acellulaire supérieure à 50 % de celle provoquée par une égale quantité de chaîne A native ;
- enfin, après administration intraveineuse unique chez le lapin à une dose voisine de 0,4 mg/kg de poids coporel, le taux plasmatique de la chaîne A à longue durée d'action (A-La) présent dans le sang circulant au temps 23 heures après l'injection est de 10 à 100 fois supérieur au taux plasmatique de la chaîne A native mesuré dans les mêmes conditions.

Disposant d'une GPIR préprarée comme décrit ci-dessus il est possible de l'utiliser pour créer des conjugués ou immunotoxines selon les procédés déjà connus.

Plus particulièrement, la présente invention a pour objet des produits, appartenant à la classe des immunotoxines (ci-après symbolysées IT), obtenues par couplage covalent entre, d'une part, un anticorps ou fragment d'anticorps, utilisé sous sa forme naturelle ou correctement modifié, possédant la capacité de reconnaître sélectivement un antigène porté par les cellules cibles visées et, d'autre part, la GPIR à longue durée d'action dite GPIR-La, obtenue comme décrit ci-dessus, le couplage entre les 2 protéines étant réalisé soit par l'intermédiaire d'une liaison disulfure, soit par l'intermédiaire d'une liaison thioéther.

Une immunotoxine formée par couplage d'un anticorps P avec une glycoprotéine inactivant les ribosomes et à longue durée d'action GPIR-La peut être représentée par la formule statistique suivante :

P' - W - GPIR-La' (I)

dans laquelle P' représente le radical d'une protéine qui est un anticorps ou un fragment d'anticorps P tel quel ou chimiquement convenablement modifié et dans lequel d'autres groupes fonctionnels sont éventuellement bloqués, GPIR-La' représente le radical d'une protéine qui est la GPIR-La telle quelle ou chimiquement convenablement modifiée et dans lequel d'autres groupes fonctionnels sont éventuellement bloqués et W représente une structure covalente bivalente contenant un groupe thioéther ou un groupe disulfure dont les atomes de soufre soit sont ceux des cystéines de P et GPIR-La, soit sont liés aux fonctions propres de P et/ou de GPIR-La par des structures d'espacement portant un groupe fonctionnel lié auxdites fonctions propres de P et/ou de GPIR-La.

Par liaison thioéther entre deux protéines on entend une liaison du type :

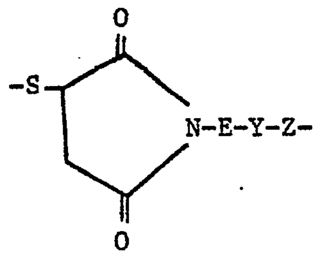

dans laquelle Z, Y et E sont tels que définis ci-dessous.

D'une façon préférentielle, la présente invention concerne une immunotoxine de formule statistique :

$$P' - W' - GPIR\text{-}La' \qquad (II)$$

dans laquelle P' et GPIR-La' sont tels que définis ci-dessus et W' représente une structure covalente choisie parmi :

a) un groupement de formule :

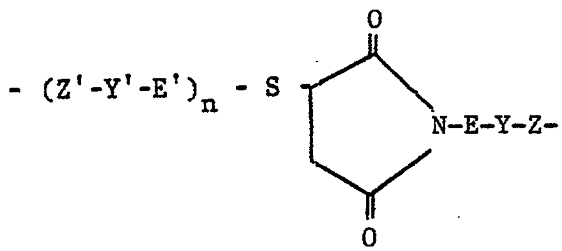

b) un groupement de formule :

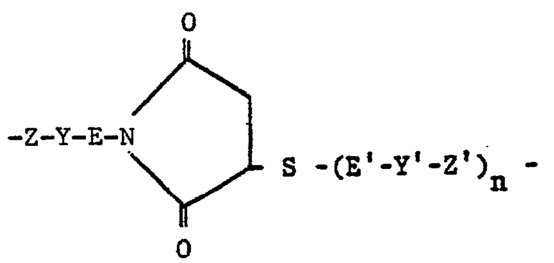

c) un groupement de formule :

$$-Z-Y-E-S-S-(E'-Y'-Z')_n-$$

d) un groupement de formule :

$$-(Z'-Y'-E')_n-S-S-E-Y-Z-$$

où

- Z et Z' identiques ou différents représentent les fonctions propres des protéines GPIR-La et P, choisies parmi l'atome d'oxygène provenant de l'hydroxyle de l'un des résidus de tyrosine ; le groupe carbonyle provenant de l'un des carboxyles terminaux ou des carboxyles libres des acides aspartiques et/ou glutamiques de GPIR-La et de P ; le groupe -NH- provenant de l'une des amines terminales de GPIR-La et de P ou de l'une des amines en position epsilon de l'un des résidus de lysine ; et seulement pour Z dans les structures covalentes (b) et (c), le groupe provenant de la structure dialdéhydique obtenue après oxydation d'une des structures glucidiques de P par l'acide periodique selon les méthodes connues.
- Y et Y' représentent des groupes engagés dans une liaison covalente avec l'une quelconque des fonctions Z et Z' des protéines GPIR-La et P.
- E et E' représentent des structures d'espacement inertes.
- n représente zéro ou 1.

Les immunotoxines de la présente invention sont représentées par les formules I et II ci-dessus, en

forme simplifiée, mais il est entendu qu'il peut y avoir plusieurs structures -W- ou -W'- liées à une même molécule P et/ou de GPIR-La et donc plusieurs GPIR-La liées à un seul P et réciproquement ; le nombre de ponts dépendant du procédé de couplage et du nombre de fonctions propres de P et de GPIR-La. Ainsi les formules statistiques I et II représentent aussi ces produits et leurs mélanges, qui répondent à la formule :

$$P' (W' - GPIR\text{-}La')_m$$

dans laquelle m est un nombre entier ou fractionnaire inférieur ou supérieur à 1.

Par exemple, si une immunotoxine est formée par couplage de la sous-unité A de la ricine native avec l'anticorps P (par exemple l'anticorps T101) par l'intermédiaire d'une structure covalente bivalente ayant un groupe disulfure dont un soufre est celui de la cystéine 257 de la chaîne A de la ricine à longue durée d'action et l'autre est lié aux oxygènes phénoliques des tyrosines de l'anticorps P par un groupe oxopropylique, elle aura la formule statistique :

$$P'(O\text{-}CO\text{-}CH_2\text{-}CH_2\text{-}S\text{-}S\text{-}A\text{-}La')_t$$

dans laquelle t représente le nombre des tyrosines de l'anticorps (par exemple de l'anticorps T101) intervenues dans le couplage.

L' immunotoxine ainsi obtenue correspond ainsi à un produit de formule II, où :

- P' est tel que défini ci-dessus, notamment le radical de l'anticorps T 101 privé de t fonctions phénoliques de ses tyrosines ;
- A-La' est le radical de la chaîne A de la ricine à longue durée d'action, privée de la fonction thiol de sa cystéine 257 ;
- W' est le groupement (c) :

$$- Z - Y - E - S - S -(E'\text{-}Y'\text{-}Z')_n\text{-}$$

où Z est l'oxygène des hydroxyles phénoliques intervenus dans le couplage, Y est -CO-, E est la structure d'espacement inerte $-CH_2\text{-}CH_2-$ et n est zéro.

Particulièrement préférés sont les immunotoxines formées par une ou plusieurs structures contenant la sous-unité A de la ricine à longue durée d'action et un seul anticorps P, représentées par la formule statistique :

$$P'(W'\text{-} A\text{-}La')_m \qquad (III)$$

dans laquelle P', W' et A-La' sont tels que définis ci-dessus et m représente le nombre de fonctions propres de la protéine P intervenues dans le couplage. Le nombre m varie de 0,3 à 12, de préférence de 0,5 à 10.

L'expression "m varie de 0,3 à 12, de préférence de 0,5 à 10" signifie que la valeur de m est statistique car dans la population des molécules d'anticorps le couplage ne se produit pas d'une façon homogène. Le nombre m peut donc ne pas être entier.

La valeur de m dépend notamment des anticorps utilisés, plus particulièrement de leur poids moléculaire.

Ainsi, si comme anticorps P de départ on utilise un fragment Fab ou Fab', la valeur de m pourra varier entre 0,3 et environ 2 ; si l'on utilise un fragment $F(ab')_2$, m pourra varier entre 0,5 et environ 4 ; pour un anticorps du type IgG, la valeur de m sera entre 0,5 et environ 6 ; enfin, pour un anticorps IgM, la valeur de m pourra varier entre 1 et environ 12.

Il est cependant préférable que le degré de substitution sur l'anticorps P soit tel qu'il conduise à une valeur de m non inférieure à 0,5 et non supérieure à 10.

Plus généralement, les structures I et II ci-dessus représentent des formules statistiques écrites de façon simplifiées, comme il a été spécifié ci-dessus.

D'une façon analogue, les formules IV, V et IX ci-dessous sont également statistiques -lorsque, le cas échéant, n est 1- car les réactifs de couplage sont préparés à partir de populations de protéines $P_1$ et $P_2$ qui ont toutes exactement les mêmes propriétés que celles prises en compte ci-dessus pour l'anticorps P, que ces protéines $P_1$ et $P_2$ soient elles-mêmes l'anticorps P ou la GPIR-La.

Selon un autre de ses aspects, la présente invention concerne un procédé pour la préparation d'une immunotoxine à longue durée d'action ayant une liaison covalente du type disulfure ou thioéther entre un anticorps et une glycoprotéine inactivant les ribosomes, caractérisé en ce que l'on forme une liaison disulfure ou thioéther entre un anticorps et une glycoprotéine inactivant les ribosomes à longue durée d'action obtenue par traitement d'une glycoprotéine inactivant les ribosomes, dont les groupes thiol sont éventuellement protégés avec une solution aqueuse d'un periodate alcalin pendant une période de temps de 0,2 à 24 h à la température de 0 à 15° C et à l'abri de la lumière en présence d'un excès d'amine primaire et par déblocage éventuel du groupe thiol.

Selon un aspect préférentiel, la présente invention concerne un procédé pour la préparation d'une immunotoxine ayant la structure I ci-dessus, caractérisé en ce qu'on fait réagir en solution aqueuse et à la

température ambiante une protéine $P_1$ qui est indifféremment soit la glycoprotéine inactivant les ribosomes à longue durée d'action GPIR-La, soit un anticorps ou fragment d'anticorps, porteuse d'au moins un groupe thiol libre attaché à ladite protéine $P_1$ directement ou par l'intermédiaire d'une structure d'espacement, avec une protéine $P_2$, différente de $P_1$, qui est indifféremment soit la glycoprotéine inactivant les ribosomes à longue durée d'action GPIR-La, soit un anticorps ou fragment d'anticorps, porteuse d'une groupement capable de couplage avec le thiol libre de la protéine $P_1$, de façon à former une liaison thioéther ou disulfure.

Selon un aspect particulièrement avantageux, la présente invention concerne un procédé pour la préparation d'une immunotoxine ayant la structure II, dans laquelle P', W' et GPIR-La' sont tels que définis ci-dessus, caractérisé en ce qu'on fait réagir en solution aqueuse et à la température ambiante une protéine de formule :

$$P_1'\text{-(Z-Y-E)}_n\text{-SH} \qquad \text{(IV)}$$

avec une protéine de formule statistique :

$$P_2'\text{-Z'-Y'-E'-G} \qquad \text{(V)}$$

où $P_1'$ et $P_2'$ représentent les radicaux des protéines $P_1$ et $P_2$ liés aux fonctions propres desdites protéines ou, seulement lorsque $P_1$ et $P_2$ sont un anticorps ou fraction d'anticorps, les radicaux des protéines $P_1$ et $P_2$ provenant de l'ouverture des structures glucidiques par action de l'acide periodique, Z, Z', Ym Y', E et E' sont tels que définis ci-dessus et G représente un groupe :

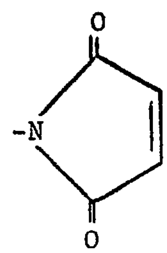

ou un groupe -S-S-X-, où X est un groupe activateur.

Tant P que GPIR-La sont donc des protéines qui présentent indifféremment :

(1) la ou les fonctions thiol qui participent au couplage et
(2) un ou plusieurs groupements fonctionnels capables de réagir avec les fonctions thiol ci-dessus pour former une liaison disulfure ou thioéther.

Selon la présente invention lesdites fonctions thiol et groupements fonctionnels sont ceux des protéines P ou GPIR-La natives, ou bien ils y sont introduits artificiellement.

La protection des groupes thiol des GPIR de départ n'est nécessaire que lorsque lesdits groupes thiol sont ceux qui seront utilisés pour le couplage avec l'anticorps. Si pour le coupalge on utilise d'autres groupes fonctionnels, par exemple l'hydroxyle phénolique des tyrosines, la protection n'est pas effectuée.

Le blocage est effectué par réaction avec un agent susceptible de substituer les fonctions SH à l'aide d'un radical qui peut être ensuite éliminé par réduction ou échange thiol/disulfure, par exemple l'acide dinitro-2,2' dithio-5,5' dibenzoïque (DTNB) ou bien par l'acide (pyridyl-2 disulfanyl)-3 propionique. En l'absence d'un tel traitement les thiols libres de la chaîne A peuvent disparaître pendant la réaction d'oxydation et de réduction en présence d'amine et dans ce cas ne peuvent être totalement régénérés par l'action d'un agent réducteur tel que le mercapto-2 éthanol. L'excès de l'agent bloquant est éliminé par dialyse.

La glycoprotéine inactivant les ribosomes dont les thiols sont bloqués est alors soumise à l'oxydation par les ions periodate et à la réduction en présence d'amines. Si par contre la sous-unité cytotoxique ne présente pas de thiol, ou bien si le ou les thiols ne servant pas au couplage, le blocage indiqué ci-dessus n'est pas mis en oeuvre.

La préparation des conjugués ou immunotoxines à partir des glycoprotéines inactivant les ribosomes et à longue durée d'action est réalisée par n'importe quel procédé convenablement choisi dans la gamme de ceux qui sont décrits dans le brevet US 4 340 535. Si la sous-unité cytotoxique choisie présente naturellement au moins un thiol la rendant apte au couplage, cette fonction sera préférentiellement mise en oeuvre par réaction avec l'anticorps ou fragment d'anticorps porteur d'un groupement disulfure activé. Si la sous-unité cytotoxique choisie ne possède pas naturellement de groupe thiol la rendant apte au couplage on pourra préférentiellement introduire artificiellement sur ladite sous-unité, après l'étape d'oxydation par les ions periodate et réduction en présence d'une amine, au moins un groupement fonctionnel porteur d'un thiol libre, selon tout procédé connu, et poursuivre le couplage comme indiqué ci-dessus.

L'introduction dudit groupement fonctionnel peut avoir lieu soit avant l'étape d'oxydation par les ions periodate et réduction en présence d'amine mais il sera alors nécessairre que le radical thiol soit bloqué pendant l'étape d'oxydation et réduction en présence d'une amine puis débloqué après cette étape, soit après l'étape d'oxydation et réduction en présence d'une amine.

Le couplage chimique entre la GPIR-La et l'anticorps (ou fragment d'anticorps) peut être réalisé selon le procédé de la présente invention par des modes opératoires qui :

- préservent les activités biologiques respectives des deux composants du conjugué : l'anticorps et la GPIR-La ;
- assurent au procédé une reproductibilité satisfaisante et un bon rendement de couplage ;
- permettent de maîtriser la valeur du rapport GPIR-La/anticorps dans le conjugué obtenu ;
- conduisent à un produit stable et soluble dans l'eau.

Parmi les modes opératoires répondant à ces caractéristiques, il faut privilégier ceux qui mettent en oeuvre une ou plusieurs fonctions thiol pour l'établissement de la laison entre les 2 protéines. En effet, ces fonctions thiol se prêtent particulièrement bien à l'établissement soit de liaisons disulfure, soit de liaisons thioéther qui satisfont les unes comme les autres aux conditions générales ci-dessus.

La préparation d'immunotoxines ayant en même temps les caractéristiques suivantes :

- la liaison covalente entre la chaîne A de la ricine et l'anticorps contient un radical disulfure,
- l'un des atomes de soufre constituant la liaison disulfure est toujours l'atome de soufre appartenant au résidu de cystéine en position 257 de la chaîne A de ricine;
- le bras de liaison réunissant la chaîne A de ricine et l'anticorps est fixé sur ce dernier au niveau de groupements $NH_2$ latéraux ou terminaux d'une chaîne peptidique.

Le couplage d'un anticorps avec la chaîne A de la ricine est décrit en détail dans le brevet US 4 340 535.

La même méthode peut être appliquée à la préparation d'immunotoxines ayant les mêmes caractéristiques et formées par couplage d'un anticorps ou fragments d'anticorps avec une GPIR-La.

La préparation d'immunotoxines formées par couplage d'un anticorps ou fragment d'anticorps avec une GPIR-La et par une liaison covalente du type disulfure ou thioéther au niveau de différents groupes fonctionnels est décrite en détail ci-après.

D'une façon générale, pour la bonne conduite des réactions de couplage entre protéines et en vue d'éliminer notamment les réticulations anarchiques, il importe que l'une des protéines à coupler et elle seule porte la ou les fonctions thiol à mettre en oeuvre, alors que l'autre protéine et elle seule porte une ou plusieurs fonctions susceptibles de réagir avec les thiols en milieu aqueux de pH compris entre 5 et 9 et à une température ne dépassant pas 30°C, pour fournir une liaison covalente stable et définie.

Les caractéristiques des protéines $P_1$ et $P_2$ utilisées comme produits de départ sont illustrées en détail ci-après. La structure d'espacement E peut être remplacée par les structures préférentielles R à $R_8$ qui ne sont données qu'à titre d'exemple.

CAS DE LA PROTEINE $P_1$

Cette protéine étant dans tous les cas celle qui porte la ou les fonctions thiol qui participeront au couplage, la situation se présente de façon diverse selon la nature de cette protéine $P_1$.

A) La protéine $P_1$ porte naturellement un ou plusieurs radicaux thiol utilisables pour permettre le couplage à la protéine $P_2$ : c'est notamment le cas si la protéine $P_1$ est le fragment d'anticorps connu sous la dénomination de F(ab)', tel qu'il est classiquement obtenu par protéolyse limitée de l'anticorps en présence de pepsine, suivie d'une réduction du (ou des) pont(s) disulfure entre chaînes lourdes.

C'est également le cas si la protéine $P_1$ est une GPIR-La, par exemple la chaîne A de la ricine modifiée (A-La), ou un dérivé de cette dernière, où l'un au moins des groupements thiol portés par les résidus de cystéine 171 et 257 de la chaîne A de ricine native est libre et accessible au couplage chimique.

Dans tous ces cas, la protéine $P_1$ porteuse de son (ou ses) groupement(s) thiol naturel(s) peut être utilisée dans cet état pour l'étape de couplage.

B) la protéine $P_1$ ne porte naturellement pas de radicaux thiol utilisables pour permettre le couplage à la protéine $P_2$ :

- c'est notamment le cas si la protéine $P_1$ est une immunoglobuline native, un anticorps entier ou un fragment d'anticorps et notamment l'un des fragments conventionnellement dénommés $F(ab)'_2$ ou F(ab),
- un autre cas où la protéine $p_1$ ne porte pas naturellement de fonction thiol utilisable pour le couplage est celui où cette protéine $P_1$ est une GPIR-La, par exemple la chaîne A de ricine à longue durée d'action où chacun des deux résidus de cystéine est soit bloqué par alkylation, soit inaccessible à la modification chimique.

Dans tous les cas, il conviendra alors d'introduire artificiellement sur de telles molécules une ou plusieurs fonctions thiol aptes à permettre le couplage.

Trois types de réaction peuvent être utilisés de préférence pour l'introduction de fonctions thiol :

a) Le premier type de réaction est l'action de l'anhydride S-acétylmercapto succinique qui est capable d'acyler des fonctions aminées de la protéine. On pourra ensuite libérer les fonctions thiol par élimination du radical acétyl protecteur, par action de l'hydroxylamine, ainsi que cela a été décrit (Archives of Biochemistry and Biophysics 1967,119 , 41-49). On pourra même, dans le cas où la (ou les) fonctions(s) thiol ainsi introduites sous forme protégée doivent réagir ultérieurement avec un radical disulfure mixte activé, se dispenser de la déprotection préalable par l'hydroxylamine ; en effet, la réaction de formation de la liaison disulfure utilisant les réactifs objets de la présente invention se produit aussi bien avec le radical S-acétyl qu'avec le thiol libre. D'autres méthodes décrites dans la littérature scientifique peuvent aussi être utilisées pour l'introduction de fonctions thiol sur la protéine à modifier.

b) Le deuxième type de réaction consiste à faire réagir la protéine par ses groupements carboxyliques avec une molécule diaminée symétrique présentant un pont disulfure, de formule :

$$H_2N - R_1 - S - S - R_1 - NH_2$$

dans laquelle $R_1$ est un groupe aliphatique comportant de 2 à 5 atomes de carbone.

La réaction se fait de préférence avec la cystamine / $R_1$ = $-(CH_2)_2$ - / en présence d'un agent de couplage tel qu'un carbodiimide et notamment un dérivé soluble dans l'eau comme l'éthyl-1 (diméthylamino-3 propyl)-3 carbodiimide, et conduit à la formation, selon les stoechiométries mises en oeuvre, de l'un des dérivés suivants ou du mélange des deux :

$$P_1'\text{-CO-NH-}R_1\text{-S-S-}R_1\text{-}NH_2 \quad \text{(Ia)}$$
$$P_1'\text{-CO-NH-}R_1\text{-S-S-}R_1\text{-NH-CO-}P_1' \quad \text{(Ib)}$$

Un tel produit de réaction peut être alors utilisé de deux manières :

-Si, dans les formules Ia ou Ib, la protéine $P_1$ est une GPIR-La, par exemple la chaîne A de ricine à longue durée d'action ou l'un de ses dérivés, le milieu réactionnel obtenu est soumis sans fractionnement à l'action d'un réducteur tel que le mercapto-2 éthanol, ce qui conduit à l'obtention d'un dérivé protéique unique de formule générale :

$$P_1'\text{-CONH-}R_1\text{-SH}$$

Le produit ainsi obtenu est alors purifié par dialyse ou filtration sur gel.

- Si, dans les formules Ia et Ib, la protéine $P_1$ est un anticorps ou l'un de ses fragements, le milieu réactionnel obtenu sera utilisé tel quel pour le couplage en utilisant alors une méthode d'échange thiol/disulfure par exemple celle décrite par Gilliland et Collier (Cancer Research 1980, 40 , 3564).

c) Le troisième type de réaction consiste à utiliser des motifs glucidiques naturellement présents sur les anticorps pour fixer le radical porteur du thiol que l'on se propose d'introduire. La protéine $P_1$ est alors soumise à une oxydation par les ions periodate selon les méthodes connues afin de faire apparaître des fonctions aldéhyde sur les motifs glucidiques. La réaction est arrêtée par addition d'un réactif qui consomme le periodate restant, par exemple un excès d'éthylèneglycol et les sous-produits sont éliminés par dialyse ou tout autre traitement approprié. Le produit obtenu est traité par une molécule diaminée symétrique présentant un pont disulfure, de formule générale :

$$H_2N\text{-}R_1\text{-S-S-}R_1\text{-}NH_2$$

dans laquelle $R_1$ est un groupe aliphatique comportant de 2 à 5 atomes de carbone. Les prcduits d'addition formés sont alors réduits en amines secondaires ou tertiaires par action d'un hydrure métallique convenable, notamment le borohydrure de sodium. La réaction se fait de préférence avec la cystamine / $R_1$ = $-(CH_2)_2$- / et conduit à la formation, selon les stoechiométries mises en oeuvre de l'un des dérivés suivants ou du mélange des deux :

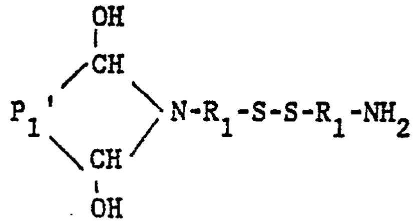

(IIa)

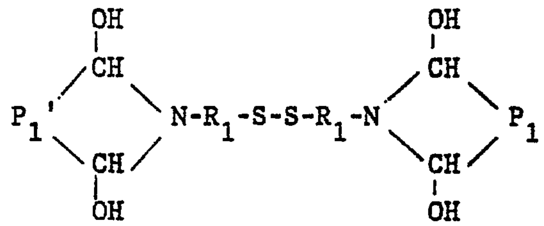

(IIb)

Le milieu réactionnel obtenu pourra alors être traité exactement comme indiqué ci-dessus pour les produits caractérisés par les structures Ia ou Ib.

Dans les deux derniers types de réaction d'introduction artificielle de groupements thiol décrits ci-dessus (ceux utilisant un réactif disulfure diaminé symétrique), il est préféreable que la protéine $P_1$ mise en oeuvre ne possède ni groupes SH libres, ni groupes aminés libres.

Dans le cas de la GPIR-La cela peut toujours être réalisé par alkylation du ou des SH naturels obtenue par réaction avec un réactif usuel des thiols tel que le N-éthylmaléiimide ou l'acide iodacétique ou l'un de ses dérivés et par méthylation des $NH_2$ naturels selon le procédé de méthylation réductive décrit par MEANS et FEENEY (Biochemistry 1968, 7, 2192). Par exemple, la chaîne A de ricine native modifiée peut présenter jusqu'à 6 radicaux méthyle par mole préalablement introduits. Une telle protéine conserve l'intégralité de ses propriétés biologiques et notamment sa capacité à inhiber la synthèse protéique ribosomale dans les cellules eucaryotes.

Dans les cas des anticorps ou fragments d'anticorps et plus généralement de toutes les substances du premier groupe tel que défini précédemment qui ne possèdent pas de groupements SH naturellement libres, il conviendra de procéder à une méthylation réductive, par exemple selon la méthode de MEANS et FEENEY : on peut ainsi habituellement introduire plusieurs dizaines de radicaux méthyle par mole d'anticorps sans modifier sa capacité à reconnaître sélectivement un antigène à la surface des cellules porteuses de cet antigène.

CAS DE LA PROTEINE $P_2$

Cette protéine est dans tous les cas celle qui porte un ou plusieurs groupements fonctionnels capables de réagir avec les thiols de la protéine $P_1$ pour former une liaison soit disulfure, soit thioéther. Ces groupements fonctionnels, toujours artificiellement introduits sur la protéine $P_2$, diffèrent selon que l'on cherche à obtenir un couplage par liaison disulfure ou par liaison thioéther et sont choisis comme indiqué ci-après.

A) Cas de la liaison disulfure.

La préparation du conjugué peut alors être représentée par le schéma :

$$P_1'\text{-(Z-Y-E)n-SH} + P_2'\text{-Z'-Y'-E'-S-S-X} \rightarrow P_1'\text{-(Z-Y-E)n-S-S-E'-Y'-Z'-}P_2' + \text{X-SH}$$

La protéine $P_2$ substituée par un atome de soufre activé est obenue à partir de la protéine $P_2$ elle-même ou de la protéine $P_2$ correctement protégée, par substitution à l'aide d'un réactif lui-même porteur d'un atome de soufre activé selon le schéma :

$$P_2 + \text{L-Y'-R-S-S-X} \rightarrow P_2'\text{-Z'-Y'-R'-S-S-X}$$

dans lequel :

-$P_2$ désigne la protéine à substituer ;

-L-Y' représente une fonction permettant la fixation covalente du réactif sur la protéine.

Le groupement fonctionnel L-Y' est une fonction capable de se lier de façon covalente avec l'une quelconque des fonctions portées par les chaînes latérales des aminoacides constitutifs de la protéine à substituer. Parmi celles-ci, on retiendra particulièrement

a) Les fonctions aminées terminales des chaînes peptidiques ou les fonctions aminées latérales des radicaux lysyle contenus dans la protéine. Dans ce cas, L-Y' pourra notamment représenter:
- un groupe carboxylique qui pourra se lier aux fonctions aminées de la protéine en présence d'un agent de couplage tel qu'un carbodiimide et notamment un dérivé soluble dans l'eau comme l'éthyl-1 (diméthylamino-3 propyl)-3 carbodiimide ; on peut à cet effet utiliser l'acide (pyridyl-2 disulfanyl)-3 propionique activé par l'éthyl-1 (diméthylamino-3 propyl)-3 carbodiimide.
- un chlorure d'acide carboxylique qui est susceptible de réagir directement avec les fonctions aminées pour les acyler ;
- un ester dit "activé" tel qu'un ester d'ortho- ou para-, nitro- ou dinitro-phényle ou encore un ester de N-hydroxy succinimide qui peut réagir directement avec les fonctions aminées pour les acyler, tel que le N-succinimidyl(pyridyl-2dithio)-3 propionate ;
- un anhydride interne d'un diacide carboxylique tel, par exemple, l'anhydride interne d'un diacide carboxylique tel, par exemple l'anhydride succinique qui réagit spontanément avec les fonctions amines pour créer des liaisons amides ;

- un groupement imidoester - 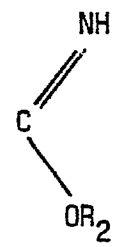 $(L = OR_2 ; Y' = -(C{=}NH)-)$

où R est un groupe alkyle, réagissant avec les groupes aminés de la protéine $P_2$ selon la réaction :

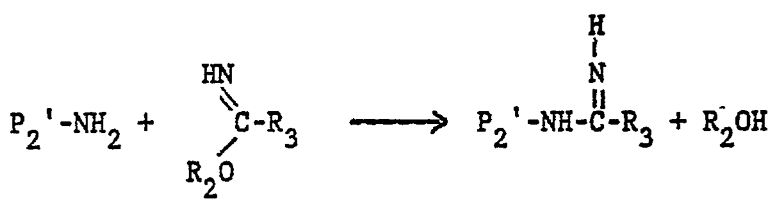

où $R_3$ représente le groupe -R-S-SX ;

b) Les fonctions phénol des radicaux tyrosyle contenus dans la protéine. Dans ce cas L-Y' pourra notamment représenter un groupement imidazolyl-1 carbonyle, réagissant avec les groupes phénol de la protéine selon la réaction :

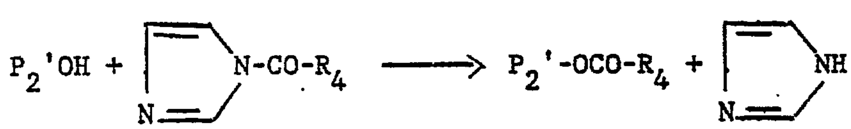

où le 1-imidazolyle est L, le groupe CO est Y' et $R_4$ est le groupe -R-S-S-X.

Le radical -S-S-X désigne un disulfure mixte activé capable de réagir avec un radical thiol libre. En particulier, dans ce disulfure mixte, X pourra désigner un groupe pyridyl-2 ou pyridyl-4, éventuellement substitué par un ou des radicaux alkyle, halogène ou carboxylique. X peut aussi désigner un groupe phényle de préférence substitué par un ou des groupes nitro- ou carboxylique. X peut encore représenter un groupe alcoxycarbonyle tel que le groupe méthoxycarbonyle.

Le radical R désigne la structure d'espacement (indiquée comme E dans la formule générale II ci-dessus) capable de porter simultanément les substituants Y' et S-S-H. Il devra être choisi de façon à ne pas comporter de fonctions susceptibles d'interférer au cours des réactions ultérieures avec les réactifs utilisés et les produits synthétisés. en particulier, le groupe R peut être un groupe $-(CH_2)_n-$ avec n compris 1 et 10, ou encore un groupe :

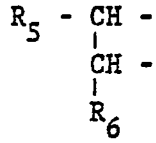

dans lequel $R_6$ désigne l'hydrogène ou groupe alkyle ayant de 1 à 8 atomes de carbone et $R_5$ désigne un substituant inerte vis-à-vis des réactifs utilisés ultérieurement tel qu'un groupe carbamate :

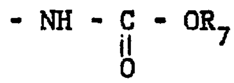

où R7 désigne un groupe alkyle droit ou ramifié ayant de 1 à 5 atomes de carbone et notamment le groupe tertiobutyle.

La réaction du composé L-Y'-R-S-S-X avec la protéine $P_2$ est effectuée en phase liquide homogène le plus souvent dans l'eau ou une solution tampon. Lorsque la solubilité des réactifs l'exige, il est possible d'ajouter au milieu réactionnel un solvant organique miscible à l'eau à une concentration finale pouvant atteindre 20 % en volume lorsqu'il s'agit d'un alcool tertiaire tel que le butanol tertiaire ou 10 % en volume lorsqu'il s'agit du diméthylformamide ou du tétrahydofuranne.

La réaction est effectuée à température ambiante pendant un temps variant de quelques minutes à quelques heures. Après quoi, une dialyse ou une filtration sur gel permet d'éliminer les produits de faible masse moléculaire et, en particulier, les excès de réactifs. Ce procédé permet d'introduire un nombre de groupements substituants par mole de protéine habituellement compris entre 1 et 15.

En utilisant de tels composés, le couplage avec la protéine $P_1$ est réalisé par mise en présence des deux protéines en solution aqueuse de pH compris entre 6 et 8, à une température ne dépassant pas 30°C, pendant un temps variant de 1 h à 24 h. La solution aqueuse obtenue est éventuellement dialysée pour éliminer les produits de faible masse moléculaire, puis le conjugé peut être purifié par diverses méthodes connues.

B) Cas de la liaison thioéther.

La préparation du conjugué consiste alors à faire réagir $P_1'$-(Z-Y-E)$_n$-SH avec la protéine $P_2$ sur laquelle auront préablablement été introduits un ou plusieurs radicaux maléiimide.

La réaction est alors représentée par le schéma suivant cité comme exemple :

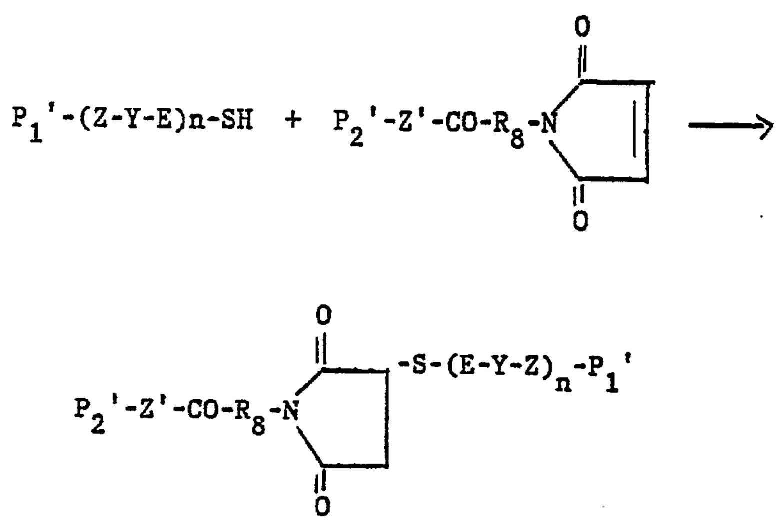

dans lequel :

- $R_8$ représente une structure d'espacement, aliphatique ou aromatique, comportant de 1 à 15 atomes de carbone, inerte vis-à-vis des réactifs utilisés ultérieurement ;
- Z' représente des groupes variables selon le type de groupement fonctionnel de la protéine $P_2$ intervenant dans le couplage.

Ainsi

. Z' = oxygène dans le cas d'un ester sur le phénol d'un résidu tyrosyle,

. Z' = NH dans le cas du couplage d'un groupement carboxylique activé sur un groupement aminé de la protéine,

. Z' = NH-$CH_2$ dans le cas de la réaction d'une chlorométhylcétone sur un groupement aminé de la protéine.

La protéine $P_2$ substituée par le ou les groupes maléiimide est obenue à partir de la protéine $P_2$ elle-même ou de la protéine $P_2$ correctement protégée, par substitution de fonctions convenables de la protéine à l'aide d'un réactif lui-même porteur du groupement maléiimide. Parmi ces fonctions convenables, on retiendra particulièrment :

a) Les fonctions aminées terminales des chaînes peptidiques ou les fonctions aminées latérales des résidus lysyle contenus dans la protéine. Dans ce cas, le réactif porteur du radical maléiimide pourra être :

- soit un réactif de formule générale :

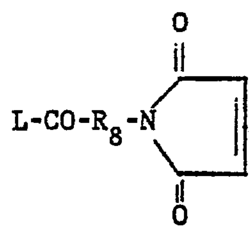

dans laquelle L-CO- représente :

. soit un groupe carboxylique, la réaction étant alors effectuée après activation de la fonction carboxylique en présence d'un agent de couplage tel qu'un carbodiimide et notamment un dérivé soluble dans l'eau tel que l'éthyl-1 (diméthylamino-3 propyl)-3 carbodiimide.

. soit un ester dit "activé" tel qu'un ester d'ortho- ou para-, nitro ou dinitrophényle, ou encore un ester de N- hydroxysuccinimide qui réagit directement avec les fonctions aminées pour les acyler.

La préparation de tels réactifs est notamment décrite dans Helvetica Chemica Acta (1975), <u>58</u> , 5312-541). D'autres réactifs de la même classe sont commercialement disponibles.

- soit un réactif de formule générale :

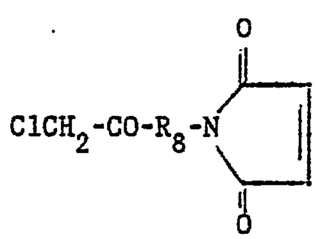

capable de réagir avec les groupements aminés de la protéine $P_2$ selon le schéma :

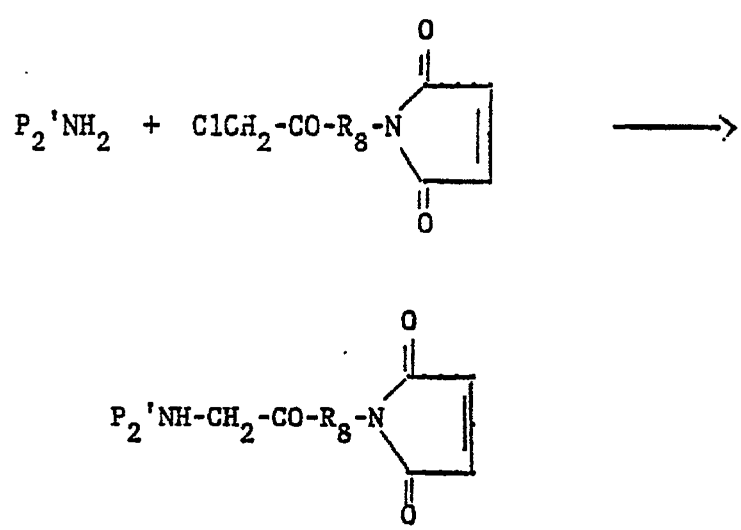

b) Les fonctions phénol des radicaux tyrosyle contenus dans la protéine. Dans ce cas, le réactif porteur du radical maléiimide pourra être un réactif de formule générale :

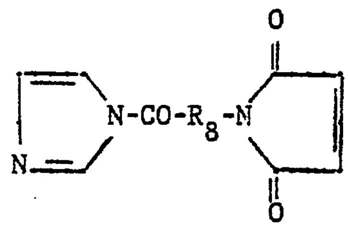

réagissant avec les groupes phénol de la protéine selon la réaction :

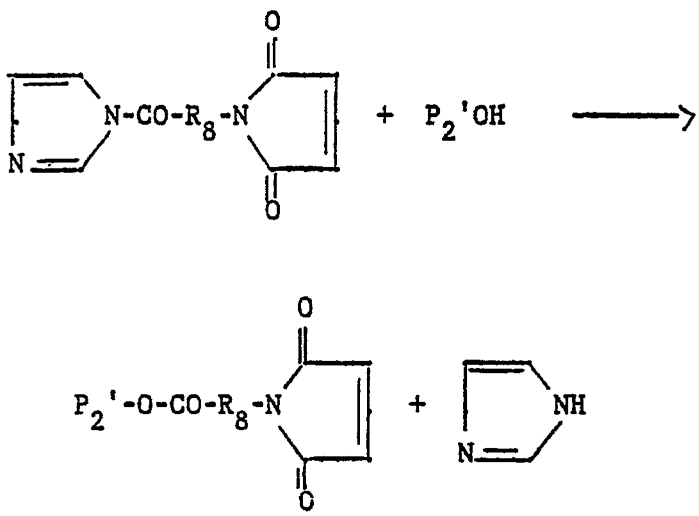

La réaction des réactifs porteurs de la maléiimide avec la protéine $P_2$ est effectuée en phase liquide homogène le plus souvent dans l'eau ou une solution tampon. Lorsque la solubilité des réactifs l'exige, il est possible d'ajouter au milieu réactionnel un solvant organique miscible à l'eau à une concentration finale pouvant atteindre 20 % en volume lorsqu'il s'agit d'un alcool tertiaire tel que le butanol tertiaire ou 10 % en volume lorsqu'il s'agit du diméthylformamide ou du tétrahydrofuranne.

La réaction est effectuée à température ambiante pendant un temps variant de quelques minutes à quelques heures. Après quoi, une dialyse ou une filtration sur gel permet d'éliminer les produits de faible masse moléculaire et, en particulier, les excès de réactifs. Ce procédé permet d'introduire un nombre de groupements substituants par mole de protéine habituellement compris entre 1 et 15.

En utilisant de tels composés, le couplage avec la protéine $P_1$ est réalisé par mise en présence des deux dérivés protéiques en solution aqueuse de pH compris entre 6 et 8, à une température ne dépassant pas 30°C, pendant un temps variant de 1 h à 24 h. La solution obtenue est éventuellement dialysée pour éliminer les produits de faible masse moléculaire, puis le conjugué peut être purifié par diverses méthodes connues.

Les composés de formule :

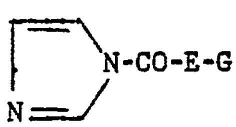 (VI)

dans laquelle E et G sont tels que définis ci-dessus, sont préparés par un procédé caractérisé en ce qu'on fait réagir un composé de formule :

G-E-COOH (VIII)

dans laquelle G et E sont tels que définis ci-dessus, avec le carbonyldiimidazole de formule :

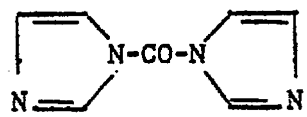

(VIII)

dans un solvant organique à la température de 10 à 40°C.

Les composés de formule VI sont particulièrement utiles comme agents de couplage sur les hydroxyles des tyrosines des protéines GPIR-La et P.

Selon un autre de ses aspects, la présente invention a pour objet de nouveaux produits ayant la formule statistique suivante :

GPIR-La"O-CO-E-G (IX)

dans laquelle :

- GPIR-La" représente le radical de la protéine GPIR-La ou toute molécule dérivant de ladite GPIR-La par modification artificielle de l'un quelconque de ses groupements fonctionnels, privée d'un ou plusieurs groupes hydroxyles phénoliques des tyrosines,
- l'atome d'oxygène est celui des groupes hydroxyles phénoliques manquant dans le radical GPIR-La",
- E et G sont tels que définis ci-dessus.

Particulièrement préférés sont les composés de formule IX dans laquelle E représente un groupe $-(CH_2)_p-$ où p est un nombre entier de 2 à 7 ou un groupe :

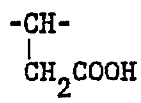

et G est un groupement de structure -S-S-X, où X est un radical activateur choisi parmi les groupes 2-pyridyle et 4-pyridyle non substitués ou substitués par un ou des halogènes ou des radicaux alkyle, carboxyle, alcoxycarbonyle, le groupe phényle non substitué ou substitué par un ou des halogènes ou des groupes nitro, alcoxy, carboxyle ou alcoxycarboxyle, ou un groupe alcoxycarbonyle.

Les produits de formule IX sont préparés en faisant réagir un produit de formule :

GPIR-La"-OH

dans laquelle GPIR-La" est tel que défini ci-dessus et le groupe hydroxyle est l'hydroxyle phénolique manquant dans les tyrosines du radical GPIR-La", avec un composé de formule IV ci-dessus à une température de 10 à 40°C dans un solvant aqueux contenant éventuellement un solvant organique miscible à l'eau, tel que par exemple un solvant éthéré comme le dioxanne ou le tétrahydrofuranne.

Dans le cas où GPIR-La est la chaîne A de ricine à longue durée d'action, les propriétés des immunotoxines IT (A-La) ainsi obtenues sont les suivantes :

- le taux de couplage moyen exprimé en nombre de moles de chaînes A modifiée par mole d'anticorps est habituellement compris entre 0,5 et 5 et en particulier entre 1 et 3 ;
- la séparation électrophorétique en gel de plyacrylamide de l'IT (A-La) conduit à une répartition du produit en une série de bandes correspondant à des produits dont les poids moléculaires diffèrent de celui de l'anticorps par incréments successifs de 30 000 daltons ;
- les études effectuées par cytofluorométrie permettent de montrer que l'anticorps n'a subi aucune altération importante au cours des réactions d'activation et de couplage auxquelles il a été soumis et qu'il reste capable au sein même du conjugué de reconnaître l'antigène contre lequel il est dirigé ;
- l'activité inhibitrice de la synthèse protéique de la chaîne A, modifiée et couplée à un anticorps, déterminée dans un modèle acellulaire en présence de mercapto-2-éthanol est totalement conservée.

L'activité cytotoxique des immunotoxines à chaîne A-La mesurée dans un test de synthèse protéique en modèle cellulaire sur des cellules présentant l'antigène cible est plus de 100 fois supérieure à celle mesurée dans les mêmes conditions pour des cellules ne présentant pas l'antigène cible. Par exemple, l'immunotoxine (désignée IT (A-La) AT15E) construite en couplant à l'aide d'un bras à pont disulfure la chaîne A-La à un anticorps monoclonal (désigné anticorps AT15E) dirigé contre l'antigène Thy 1.2 présent à la surface de certaines cellules leucémiques de souris, est environ 1.000 fois plus toxique vis-à-vis des cellules Thy 1.2 positives que des cellules Thy 1.2 négatives.

Enfin, après administration intraveineuse de l'IT (A-La) chez le lapin à une dose voisine de 0,4 mg/kg de poids corporel exprimé en chaîne A, le taux plasmatique de l'IT (A-La) présent dans le sang circulant au temps 24 h après l'injection est de 10 à 200 fois supérieur au taux plasmatique de l'IT classique mesuré dans les mêmes conditions. Ainsi typiquement chez le lapin, il apparaît que les taux plasmatique de l'IT (A-La) AT15E dans le sang circulant au temps 24 h après l'injection est de 10 % du produit présent au temps zéro contre 0,08% pour l'IT AT15E classique correspondante à la chaîne A non modifiée, au même temps, soit un accroissement d'un facteur 120 environ.

On obtient ainsi des immunotoxines modifiées qui ont acquis un caractère nouveau quant à leurs propriétés pharmacocinétiques.

En particulier, par modification appropriée de la sous-unité cytotoxique on a pu ajouter aux propriétés de cytotoxicité spécifique des immunotoxines, sans qu'il leur soit porté atteinte, une nouvelle propriété tout aussi intrinsèque : la capacité à manifester une lente cinétique d'élimination plasmatique après injection chez les animaux supérieurs ou chez l'homme.

La présente invention a également pour objet les compositions pharmaceutiques anticancéreuses contenant, à titre de principe actif, une immunotoxine modifiée selon l'invention, en combinaison avec un véhicule pharmaceutiquement acceptable, approprié pour l'administration par voie injectable en particulier par voie intraveineuse.

Les exemples suivants permettent de mieux comprendre l'invention sans en limiter la portée.

EXEMPLE 1:

Cet exemple démontre, après injection intraveineuse chez l'animal, l'élimination lente de la chaîne A de ricine modifiée par réaction d'oxydation par le periodate de sodium réalisée en présence d'un excès de L-leucine.

Modification de la chaîne A de ricine par action simultanée du periodate de sodium et de la L-leucine.

a) Blocage du SH naturel de la chaîne A par le DTNB.

La chaîne A de ricine a été préparée et purifiée selon le mode opératoire décrit dans le brevet US 4 340 535.

A 23 ml d'une solution de chaîne A de ricine comportant 0,84 groupement thiol par mole de chaîne A à 7,2 mg/ml dans le tampon PBS (tampon phosphate 20 mM, NaCl 150 mM pH 7), on ajoute 20 équivalents d'acide dinitro-2,2′ dithio-5,5′ dibenzoïque (DTNB) sous la forme de 1 ml de solution de DTNB dans un tampon phosphate 125 mM, cette solution étant amenée à pH 7 à l'aide d'hydroxyde de sodium. On dialyse ensuite la solution contre du PBS à 4°C et on obtient ainsi 162 mg de chaîne A bloquée sur la fonction thiol, en solution à 6,9 mg/ml.

b) Oxydation periodique et formation de base de Schiff avec la L-leucine sur la chaîne A bloquée sur la fonction thiol.

A 79,4 mg de chaîne A bloquée sur la fonction thiol contenus dans 11,5 ml de solution amenée à pH 6,5 à l'aide d'acide acétique, on ajoute 12 ml d'une solution de L-leucine à 52 mg/ml dans un tampon phosphate 30 mM pH 6,5 puis 0,5 ml d'une solution de periodate de sodium 0,5 M dans l'eau. On laisse l'incubation se poursuivre pendant 17 heures à 4°C dans l'obscurité. La réaction est arrêtée par addition de 2,8 ml d'une solution aqueuse d'éthylèneglycol 1 M. Après 15 minutes d'incubation à 20°C, le milieu réactionnel est dialysé à 4°C contre du tampon PBS pendant 48 h. Après centrifugation à 10.000 xg pendant 30 minutes, on obtient 28 ml (à 1,9 mg/ml) de chaîne A bloquée sur la fonction thiol et modifiée sur ses résidus osidiques par oxydation et formation de base de Schiff.

c) Déblocage des groupements thiol.

25 ml de solution à 1,9 mg/ml de chaîne A bloquée et modifiée sur ses résidus osidiques sont ramenés à 10,5 ml par concentration, puis on ajoute 0,214 ml de mercapto-2 éthanol à 50 %. On laisse l'incubation se poursuivre pendant 1 h à 20°C. On dialyse ensuite la solution contre le tampon PBS à 4°C. On obtient ainsi 26,5 mg de chaîne A modifiée à une concentration de 2.65 mg/ml.

En utilisant la technique au DTNB (Methods in Enzymology, 1972, 25 , 457 (Academic Press)), on détermine que la chaîne A modifiée obtenue présente 0,89 groupement thiol libre par mole. Le poids moléculaire de la chaîne A modifiée est de 30.000± 3.000, déterminé par électrophorèse en gradient de polyacrylamide en présence de dodécyl-sulfate de sodium.

La préparation de chaîne A dont les motifs osidiques ont été modifiés, obtenue précédemment, a été étudiée en ce qui concerne ses activités enzymatiques d'inhibition de la synthèse protéique et ses propriétés pharmacocinétiques.

B) Activité enzymatique de la chaîne A-La à longue durée d'action, mesurée sur un modèle acellulaire.

La propriété biologique fondamentale de la chaîne A de ricine est d'inhiber la synthèse protéique cellulaire par altération de la sous-unité ribosomale 60S.

Le protocole in vitro utilisé comporte l'emploi de fractions subcellulaires de foie de rat convenablement complémentées et capables d'incorporer la 14C-phénylalanine en présence d'un ARN messager artificiel, l'acide polyuridylique.

Le mode opératoire employé pour préparer les fractions subcellulaires et pour mesurer l'incorporation de 14C-phénylalanine est une adaptation de la méthode décrite dans Biochemica Biophysica Acta, 1973, 312 , 608-615, mettant en oeuvre à la fois une fraction microsomale et une fraction de cytosol des hépatocytes de rat. L'échantillon contenant la chaîne A est introduit sous la forme d'une solution convenablement diluée dans un tampon Tris HCl 50 mM, pH 7,6, contenant du mercapto-2 éthanol à 0,2 % et de la sérum-albumine bovine à 15 microgrammes/ml.

A partir des données de comptage, on calcule par rapport à un milieu témoin sans inhibiteur le pourcentage d'inhibition de l'incorporation de 14C-phénylalanine dans les protéines pour chaque milieu réactionnel contenant de la chaîne A de ricine.

Les courbes obtenues permettent de calculer la $CI_{50}$ ou concentration de chaîne A (native ou modifiée) qui inhibe de 50 % l'incorporation du précurseur radiomarqué dans les protéines. On observe ainsi une $CI_{50}$ égale à $3.10^{-10}$ mole/l pour la chaîne A-La modifiée et bloquée. La $CI_{50}$ de la chaîne A témoin de l'expérience est de $10^{-10}$ mole/l : compte tenu de la précision des mesures, la modification n'entraîne donc pas de perte significative d'activé de la chaîne A.

C) Propriétés pharmacocinétiques de la chaîne A à longue durée d'action modifiée sur ses motifs osidiques (chaîne A-La).

La chaîne A (native ou modifiée) est administrée chez le lapin par injection unique dans une veine de l'oreille. La qunatité de chaîne A injectée correspond à 0,415 mg/kg. Les échantillons de sang sont prélevés au cours du temps sur héparine. Les plasmas sont analysés à l'aide d'un test immunoradiométrique ci-après désigné par l'abréviation IRM-1.

Cette technique a l'avantage de doser la chaîne A sans modification de celle-ci. Ce dosage est réalisé dans des plaques à microtitrations (par exemple : "NUNC-TSP® screening system" Poly Labo Block France) dont le couvercle est muni de pointes hyperabsorbantes qui plongent dans les puits du socle. Ces pointes constituent les phases solides. Des anticorps de brebis anti-chaîne A de ricine (ci-après désignés sous l'abréviation Acl), purifiés par chromatographie d'affinité, sont absorbés sur les phases solides. Pour cela 200 microlitres d'une solution d'Acl à 10 microgrammes/ml dans le tampon phosphate PBS sont distribués dans les puits. Les pointes sont mises en contact d'abord avec la solution d'Acl pendant 24 h à 4°C puis avec du sérum de veau foetal pendant 3 h à 20°C pour saturer tous les sites de fixation. L'immunoabsorbant saturé est alors mis en contact pendant 3 h à 20°C avec les échantillons plasmatiques à doser à différentes dilutions ou avec des solutions de chaîne A de concentrations connues pour l'établissement de la courbe d'étalonnage. Un lavage est effectué par un tampon PBS, puis l'immunoabsorbant est mis en contact pendant 2 h à 20°C avec les anticorps de brebis antichaîne A de ricine purifiée par chromatographie d'affinité et radiomarqués (ci-après désignés par l'abréviation Ac2). Le radiomarquarge de l'Ac2 est réalisé avec l'iode 125 en présence de chloramine T selon la méthode de Greenwood et Hunter (Biochem. J., 1963, 89 , 114) ; l'activité spécifique des Ac2 radiomarqués est de $1.85$ à $3.7 \times 10^5$ Bq/µg (5 à 10 microcuries/microgramme). $10^6$ coups par minute (cpm) d'Ac2 radiomarqués sont introduits sous 200 microlitres dans le tampon PBS, contenant 0,1 % de sérum-albumine bovine. La mesure de la concentration en chaîne A dans les échantillons à doser se fait par référence à la courbe d'étalonnage réalisée avece la chaîne A introduite à différentes concentrations connues. Lorsque de la chaîne A-La à longue durée d'action est injectée chez l'animal, cette même chaîne A-La est utlisée pour l'établissement de la courbe d'étalonnage correspondante.

Les valeurs de concentration en chaîne A (native ou modifiée) dans le plasma sanguin mesurées par cette technique sont reproductibles et fiables. Le seuil de détection est de 1 nanogramme/ml. L'étude de la reproductibilité intra- et inter-essais donne des coefficients de variation inférieurs à 10 % pour les valeurs de concentrations comprises dans la gamme de 1 à 200 nonogrammes/ml.

Les résultats de ces expériences sont représentés sous la forme de courbes présentant en abscisse le temps, exprimé en heures, et en ordonnée, sur échelle logarithmique, la concentration plasmatique du produit testé, rapportée en pour-cent de la concentration plasmatique théorique au temps zéro. Cette valeur appelée "concentration plasmatique relative" (CPR) est calculée à l'aide de l'expression suivante :

$$CPR = \frac{\text{Concentration mesurée au temps t}}{\text{Quantité injectée/volume plasmatique}} \times 100$$

Le volume plasmatique est considéré égal à 36 ml/kg de poids corporel de l'animal.

La figure 1 montre les courbes d'élimination plasmatique, en fonction du temps après injection intraveineuse, de la chaîne A de ricine native et de la chaîne A-La modifiée. Pour la chaîne A native (courbe 1) il apparaît deux phases : dans la première phase, le produit disparaît très rapidement de la circulation, puisque trois heures après l'injection il ne reste plus dans le plasma que 0,1 % de la dose administrée. Dans la deuxième phase, la décroissance est plus lente.

Lorsque la chaîne a été modifiée sur ses motifs osidiques (chaîne A-La, courbe 2), le profil d'élimination est profondément modifié : la première phase d'élimination, responsable de la disparition de la plus grande partie du produit, est pratiquement abolie, ce qui conduit à un accroissement considérable des taux plasmatiques de sous-unité cytotoxique active. vingt heures après l'injection, la concentration de la chaîne A-La modifiée est 460 fois supérieure à la concentration de la chaîne a non modifiée (courbe 2).

Ainsi, ces résultats prouvent que les réactions d'oxydation par le periodate de sodium et de blocage par formation de base de Schiff par la L-leucine ont modifié les sucres impliqués dans le processus de reconnaissance responsable de l'élimination de la chaîne A au point d'empêcher cette reconnaissance sans que l'activité biologique caractéristique de la chaîne A soit altérée.

EXEMPLE 2:

Cet exemple démontre, après injection intraveineuse chez l'animal, l'élimination lente de la chaîne A de ricine modifiée par réaction d'oxydation par le periodate de sodium et par formation de base de Schiff en présence de L-alanine, ou d'acide L-glutamique ou de L-arginine.

A) Mode opératoire.

La chaîne A est modifiée selon le procédé décrit dans l'exemple 1 excepté que la réaction de Schiff est réalisée en présence soit d'acide glutamique, soit d'arginine, soit d'alanine, à la place de la leucine, Les quantités de chacun des ces acides aminés introduites sont $10^3$, 5 $10^3$ et $10^4$ fois supérieures aux quantités molaires de chaîne A mises en oeuvre, pour l'acide glutamique, l'arginine, et l'alanine, respectivement.

B) Résultats.

Le tableau I montre les propriétés de ces différentes chaînes A-La modifiées. Les résultats obtenus avec la chaîne A native et avec la chaîne A-La modifiée par le periodate de sodium et la L-leucine tel que cela est décrit dans l'exemple 1 sont rappelés à titre comparatif.

TABLEAU I

| | Amines primaires utilisées pour la formation des bases de Schiff | | | | | |
|---|---|---|---|---|---|---|
| | Chaîne A native | L-leucine | acide glutamique | L-arginine | L-alanine |
| Inhibition de synthèse protéique en modèle acellulaire ($CI_{50}$ x $10^{-10}$M) | 10 | 3 | 3,4 | 2 | 3,3 |
| Thiol libre par chaîne A | 0,9 | 0,89 | 0,87 | 0,8 | 0,85 |
| Poids moléculaire (± 3.000) | 30.000 | 30.000 | 30.000 | 30.000 | 30.000 |
| Concentration plasmatique relative (%) 24 h après injection | 0,01 | 7 | 7 | 8,5 | 10 |

Ainsi, ces résultats prouvent que les réactions d'oxydation par le periodate de sodium accompagnées par le bloquage simultané des groupes aldéhyde sous forme de bases de Schiff par la L-alanine, la L-arginine ou l'acide L-glutamique ont modifié les sucres impliqués dans le processus de reconnaissance responsable de l'élimination biologique de la chaîne A in vivo au point d'empêcher l'essentiel de cette reconnaissance, sans que la propriété d'inactivation des ribosomes de la chaîne A soit significativement altérée et sans perte des groupements thiol utilisables pour couplages ultérieurs.

EXEMPLE 3 :

Immunotoxine (abrégé IT) obtenue entre un anticorps anticellules T de souris (anticorps dirigé contre l'antigène Thy 1.2) substitué par des groupements disulfure activé et la chaîne A-La de ricine :

a) Anticorps anti-cellules T de souris ou anticorps AT15E :

Cet anticorps a été obtenu selon la méthode décrite dans Journal of Immunology, 1979, 122 , 2491-2498.

b) Chaîne A-La de ricine :

La chaîne A-La de ricine utilisée a été préparée ainsi qu'il a été indiqué dans l'exemple 1.

c) Anticorps activé anti-cellules T de souris :

A 43,7 ml d'une solution à 7 mg/ml d'anticorps dans un tampon phosphate 125 mM, pH 7, on ajoute 3,62 mg de N-succinimidyl (pyridyl-2 dithio)-3 propionate dans l'éthanol à 95 % sous un volume de 0,326 ml. Le mélange est agité 30 minutes à 20°C. Après dialyse contre du tampon phosphate 125 mM, pH 7, la solution protéïque est centrifugée à 10.000 xg pendant 30 minutes à 4°C, et l'on obtient ainsi 96,8 mg d'anticorps activé à une concentration de 2,42 mg/ml. Par dosage spectrophotométrique à 343 nm de pyridine-thione-2 libérée par échange avec le mercapto-2 éthanol, on constate que l'on a obtenu un anticorps portant 6,7 groupements disulfure mixte activé par mole d'anticorps.

d) Préparation de l'immunotoxine à chaîne A-La de ricine à longue durée d'action.

A 14,8 ml de la solution d'anticorps activé précédemment obtenue (concentration 2,42 mg/ml soit 35,8 mg d'anticorps activés) on ajoute 10 ml de chaîne A-La à 2,65 mg/ml obtenue comme indiqué à l'exemple 1 et on incube 20 heures à 25°C. La solution est centrifugée puis purifiée par filtration sur gel (gel AcA 44) avec mesure de la densité optique de l'effluent à 280 nm. Le regroupement des fractions contenant à la fois l'anticorps et la chaîne A modifiée conduit à 52 ml de solution d'immontoxine à 0,315 mg/ml soit 18,25 mg. Cette solution contient 0,076 mg de chaîne A-La modifiée couplée à l'anticorps par ml. Le taux de couplage moyen de cette préparation est donc de 1,6 mole de chaîne A-La par mole d'anticorps.

L'immunotoxine à chaîne A-La de ricine obtenue comme indiqué ci-dessus a été étudiée en ce qui concerne ses propriétés pharmacocinétiques et ses propriétés de cytotoxicité spécifique vis-à-vis des cellules cibles.

EXEMPLE 4

Cet exemple démontre l'acquisition de la propriété de lente élimination plasmatique des immunotoxines à chaîne A de ricine à longue durée d'action désignées en abrégé IT (A-La).

A) Mode opératoire.

Le conjugué préparé selon le procédé décrit dans l'exemple 3 est administré chez le lapin par injection unique dans une veine de l'oreille. La quantité injectée correspond à 0,415 mg/kg exprimé en chaîne A. Les échantillons de sang sont prélevés au cours du temps sur héparine. Les plasmas sont analysés à l'aide d'un test immunoradiométrique à deux sites ci-après désigné par l'abréviation IRM-3

Cet essai est réalisé selon la même technique que pour le test IRM-1 (décrit dans l'exemple 1) excepté que la solution Ac2 est ici une solution d'anticorps de chèvres anti-IgG de souris purifiés par chromatographie d'affinité et radiomarqués comme décrit pour la technique IRM-1 (décrit dans l'exemple 1). La mesure de la concentration en immunotoxine modifiée dans les échantillons à doser se fait par référence à une courbe d'étalonnage réalisée avec l'immunotoxine modifiée introduite à différentes concentrations connues. L'essai IRM-3 répond aux mêmes caractéristiques de fiabilité et de reproductibilité que celles décrites pour la technique IRM-1.

Pour comparaison, une étude contrôle est réalisée dans les mêmes conditions avec le conjugué appelé IT-AT15E obtenu par réaction entre le même anticorps AT15E substitué par des groupements disulfure activé et la chaîne A native de ricine. La préparation est réalisée selon la même procédure que celle décrite dans l'exemple 3 de ce conjugué. Les résultats de ces expériences de pharmacocinétique sont représentés de la même façon que pour la chaîne A de ricine non couplée dans l'exemple 1.

B) Résultats.

La figure 2 montre les courbes d'élimination plasmatique en fonction du temps de l'IT AT15E (courbe 1) et de l'IT (A-La) AT15E (courbe 2) injectées par voie intraveineuse. vingt-quatre heures après l'injection, la concentration d'immunotoxine active est 120 fois supérieure pour l'IT (A-La) AT15E que pour l'IT AT15E classique. Ce fait démontre que les nouvelles propriétés pharmacocinétiques de la chaîne A-La oxydée et bloquée sont conservées après couplage à un anticorps.

EXEMPLE 5 :

Cet exemple démontre le maintien des propriétés de cytotoxicité spécifique de l'IT (A-La) AT15E vis-à-vis des cellules cibles Thy 1.2 positives.

La propriété biologique fondamentale de la chaîne A de ricine est d'inhiber la sythèse protéique cellulaire par altération de la sous-unité ribosomale 60S.

La technique utilisée met en oeuvre un modèle cellulaire dans lequel on mesure l'effet des substances étudiées sur l'incorporation de 14C-leucine dans les cellules cancéreuses en culture.

Les cellules utilisées appartiennent à la lignée cellulaire T2 issue d'une leucémie T qui porte l'antigène Thy 1.2. Les cellules sont incubées en présence de la substance à étudier puis, en fin d'incubation, on procède à la mesure du taux d'incorporation de 14C-leucine par les cellules ainsi traitées.

Cette mesure est effectuée selon une technique adaptée de la technique décrite dans Journal of Biological Chemistry, 1974 249 (11), 3557-3562 utilisant le traceur 14C-leucine pour la détermination du taux de synthèse protéique. La détermination de la radioactivité incorporée est effectuée ici sur les cellules entières isolées par filtration.

A partir de ces déterminations, on peut tracer les courbes effets/doses présentant en abscisse la concentration molaire en chaîne A des substances étudiées et en ordonnée l'incorporation de 14C-leucine exprimée en pourcentage de l'incorporation des cellules témoins en l'absence de toute substance affectant la synthèse protéique.

On peut aussi déterminer pour chaque substance étudiée la concentration qui inhibe 50 % de l'incorporation de 14C-leucine ou "concentration inhibitrice 50" ($CI_{50}$).

Le tableau II ci-après représente les valeurs de $CI_{50}$ obtenues dans la même expérience avec l'IT-(A-La)-AT15E et IT AT15E d'une part, la chaîne A native et la chaîne A-La non couplée, d'autre part.

On peut constater que l'IT (A-La) AT15E a une très forte activité cytotoxique, identique à celle obtenue avec l'immunotoxine à chaîne A native correspondante et qui est environ 1.000 fois supérieure à celle de la chaîne A-La modifiée non couplée, mesurée dans les mêmes conditions.

TABLEAU II

| Produit testé | Concentration inhibitrice 50 |
|---|---|
| IT (A-La) AT15E | $2.10^{-10}$M |
| IT AT15E | $2.10^{-10}$M |
| Chaîne A native | $3.10^{-7}$M |
| Chaîne A-La | $2.10^{-7}$M |

EXEMPLE 6 :

Cet exemple démontre, après injection intraveineuse chez l'animal,

1) l'élimination rapide de la gélonine native et
2) l'élimination lente de la gélonine modifiée par réaction d'oxydation par le periodate de sodium réalisée en présence de L-leucine.

A) Modification de la gélonine par action sumultanée du periodate de sodium et de la L-leucine.

La gélonine a été préparée et purifiée à partir de Gélonium multiflorum selon la méthode décrite (J. Biol. Chem., 1980, 255 , 6947-6953). La réaction de modification est réalisée dans les mêmes conditions que celles décrites pour la chaîne A de ricine dans l'exemple 1 excepté que l'étape de blocage des thiols par le DTNB n'est pas pratiquée.

En effet, le couplage de la gélonine à l'anticorps n'étant pas généralement pratique à partir des groupements thiols naturels de la gélonine, les groupements thiols seront introduits artificiellement après l'étape d'oxydation, selon la technique décrite dans Cancer Res., 1984, 44 , 129-133. A 1 ml de solution à 3 mg/ml de gélonine dans le tampon PBS, amenée à pH 6,5 à l'aide d'acide acétique 1M, on ajoute 1 ml d'une

solution de L-leucine à 52 mg/ml dans un tampon phosphate 30 mM pH 6,5 puis 40 microlitres d'une solution de periodate de sodium à 0,5 M dans l'eau. On laisse l'incubation se poursuivre pendant 17 h à 4°C dans l'obscurité. La réaction est arrêtée par addition de 210 microlitres d'une solution aqueuse d'éthylèneglycol 1 M. Après 15 minutes d'incubation à 20°C, le milieu réactionnel est dialysé à 4°C contre du tampon PBS. On obtient ainsi après centrifugation à 10,000 x g pendant 30 minutes 2,9 mg de gélonine oxydée à une concentration de 2,5 mg/ml.

Comme la chaîne A de ricine, la propriété fondamentale de la gélonine est d'inhiber la synthèse protéique de cellules eucaryotes par altération de la sous-unité ribosomale 60S (Biochem. J., 1982, 207 , 505-509). Dans le cas de la gélonine aussi, la modification n'entraîne pas de perte significative d'activité.

B) Propriétés pharmacocinétiques de la gélonine à longue durée d'action.

La gélonine native ou modifiée selon les procédures développées ci-dessus est administrée chez le lapin par injection unique dans une veine de l'oreille. La quantité de gélonine injectée est comprise entre 0,3 et 0,4 mg/kg. Les échantillons de sang sont prélevés au cours du temps sur héparine. Les plasmas sont analysés à l'aide d'un test immunoradiométrique ci-après désigné par l'abréviation IRM-2.

Cet essai est réalisé selon la même technique que pour le test IRM-1 excepté que la solution Ac1 est ici une solution d'anticorps de lapin anti-gélonine purifiés par chromatographie d'affinité, les anticorps Ac2 étant les mêmes anticorps radiomarqués. La procédure de radiomarquage est identique à celle décrite pour la technique IRM-1. La mesure de la concentration en gélonine native ou gélonine modifiée dans les échantillons à doser se fait par référence à une courbe d'étalonnage réalisée avec la gélonine native ou modifiée introduite à différentes concentrations connues. L'essai IRM-2 répond aux mêmes caractéristiques de fiabilité et reproductibilité que celles décrites pour la technique IRM-1.

Les courbes d'élimination plasmatique, en fonction du temps, de la gélonine native et de la gélonine modifiée injectées par voie intraveineuse, montrent que la gélonine native, comme la chaîne A de ricine native, disparaît très rapidement de la circulation puisque 99,99 % de la gélonine présente dans le sang circulant disparaissent en 24 h. Lorsque la gélonine a été modifiée sur ses motifs polysaccharidiques, le profil d'élimination est profondément modifié : 24 h après l'injection, la concentration de la gélonine oxydée est environ 300 fois supérieure à celle de la gélonine native.

Ainsi, comme pour la chaîne A de ricine, ces résultats prouvent que les réactions d'oxydation par le periodate de sodium et de blocage par formation de base de Schiff par la L-leucine, ont modifié les sucres impliqués dans le processus de reconnaissance responsable de l'élimination de la gélonine au point d'empêcher cette reconnaissance.

Ces immunotoxines modifiées peuvent être utilisées pour le traitement des affections cancéreuses ou non dont les cellules cibles seraient reconnues par l'anticorps utilisé pour la préparation de l'imminotoxine. Les modalités optimales d'administration ainsi que la durée du traitement devront être déterminées dans chaque cas en fonction du sujet et de la nature d l'affection à traiter. Les nouveaux médicaments selon l'invention sont conditionnés pour être utilisés par voie injectable et de préférence par voie intraveineuse.

**Revendications pour les états contractants: BE, CH, DE, FR, GB, IT, LI, LU, NL, SE**

1. GPIR (Glycoprotéine inactivant les robosomes) modifée, ayant l'activité inhibant les ribosomes de la GPIR native et une durée d'action in vivo prolongée par rapport à celle de la GPIR caractérisée en ce qu'elle est obtenue par traitement d'une GPIR par des ions périodate en présence d'une amine primaire en excès.

2. GPIR modifée selon la revendication 1caractérisée en ce qu'au moins l'un des groupes thiol de la GPIR est protégé pendant le traitement par les ions periodate.

3. GPIR modifiée selon l'une des revendications 1 ou 2, caractérisé en ce que le traitement par les ions periodate a lieu dans une solution aqueuse de pH 5 à 7, à une température de 0 à 15° C, à l'obscurité et pendant une période de 0,2 à 24 heures.

4. GPIR modifiée selon l'une quelconque des revendications 1 à 3, caractérisé en ce que la solution réactionnelle contient de 1 à 10 mg/ml de chaîne a réactive, 10 à 50 mM de periodate alcalin et 50 à 50 mM d'amine primaire.

5. GPIR modifiée selon l'une quelconque ds revendications 1 à 4, caractérisée en ce que l'amine primaire est une amine aliphatique choisi parmi les alkylamines, les aminoacides et les peptides hydrosolubles.

6. GPIR modifiée selon la revendication 5, caractérisée en ce que l'aminoacide est choisi parmi les racémiques ou les énantiomères de glycine, alanine, valine, leucine, isoleucine, arginine, acide aspartique, acide glutamique.

7. Procédé de préparation d'une GPIR modifiée selon l'une quelconque des revendications 1 à 6, caractérisé en ce qu'on fait réagir sur une GPIR en solution aqueuse un excès de periodate alcalin et d'une amine primaire.

8. Procédé selon la revendication 7, caractérisée en ce que le traitement par les ions periodate a lieu dans une solution aqueuse de pH 5 à 7, à une température de 0 à 15°C, à l'obscurité et pendant une période de 0,2 à 24 heures.

9. Procédé selon l'une des revendications 7 ou 8, caractérisé en ce que la solution réactionnelle contient de 1 à 10 mg/ml de GPIR réactive, 10 à 50 mM de periodate alcalin et 50 à 500n mM d'amine primaire.

10. Procédé selon l'une quelconque des revendications 7 à 9, caractérisé en ce qu'en outre avant la réaction d'oxydation on traite la GPIR par un réactif classique de protection des groupes SH, et que l'on libère les groupes SH après le traitement par les ions $IO_4$- et l'amine primaire.

11. Procédé selon la revendication 10, caractérisé en ce que le réactif de protection des groupes SH est choisi parmi l'acide dinitro-2,2' dithio-5,5' dibenzoïque et l'acide (pyridyl-2 disulfanyl)-3 propionique et en ce que la déprotection des groupes SH est effectuée par action du mercapto-2 éthanol.

12. Immunotoxine à durée d'action prolongée in vivo, caractérisée en ce qu'elle résulte du couplage d'un anticorps ou d'un fragment d'anticorps à une GPIR modifiée selon l'une quelconque des revendications 1 à 6.

13. Imunotoxine selon la revendication 12, caractérisée en ce que le couplage entre l'anticorps ou le fragment d'anticorps et la GPIR modifiée a lieu par l'intermédiaire d'un pont disulfure.

14. Immunotoxine selon la revendication 13, caractérisée en ce que le couplage est effectué avec un réactif hétérobifonctionnel choisi parmi le N-succinimidyl (pyridyl-2 dithio)-3 propionate ou l'acide (pyridyl-2-disulfanyl)-3 propionique activé par l'éthyl-1 (diméthylamino-3 propyl)-3 carbodiimide.

15. Composition pharmaceutique anticancéreuse caractérisée en ce qu'elle contient, à titre de principe actif, une immunotoxine selon l'une quelconque des revendications 12 à 14.

**Revendications pour les états contractants: AT, ES, GR**

1. Procédé pour l'obtention d'une GPIR (Glycoprotéine inactivant les ribosomes) modifiée, ayant l'activité inhibant les ribosomes de la GPIR native et d'une durée d'action in vivo prolongée par rapport à celle de la GPIR, caractérisé en ce qu'il consiste à traiter une GPIR par des ions periodate en présence d'une amine primaire en excès.

2. Procédé selon la revendication 1, caractérisé en ce qu'au moins l'un des groupes thiol de la GPIR est protégé pendant le traitement par les ions periodate.

3. Procédé selon l'une des revendications 1 ou 2 caractérisé en ce que le traitement par les ions periodate à lieu dans une solution aqueuse de pH 5 à 7, à une température de 0° à 15° C, à l'obscurité et pendant une période de 0,2 à 24 heures.

4. Procédé selon l'une quelconque des revendications 1 à 3, caractérisé en ce que la solution réactionnelle contient de 1 à 100 mg/ml de chaîne A réactive, 10 à 50 mM de periodate alcalin et 50 à 500 mM d'amine primaire.

5. Procédé selon l'une quelconque des revendications 1 à 4, caractérisé en ce que l'amine primaire est une amine aliphatique choisie parmi les alkylamines, les aminoacides et les peptides hydrosolubles.

6. Procédé selon la revendication 5, caractérisé en ce que l'aminoacide est choisi parmi les racémiques ou les énantiomères de glycine, alanine, valine, leucine, isoleucine, arginine, acide aspartique, acide glutamique.

7. Procédé selon l'une quelconque des revendications 1 à 6, caractérisé en ce qu'en outre avant la réaction d'oxydation on traite la GPIR par un réactif classique de protection des groupes SH et que l'on libère les groupes SH après le traitement par les ions $IO_4$- et l'amine primaire.

8. Procédé selon la revendication 7, caractérisé en ce que le réactif de protection des groupes SH est choisi parmi l'acide dinitro-2.2' dithio-5,5' dibenzoïque et l'acide (pyridiyl-2 disulfanyl)-3 propionique et en ce que la déprotection des groupes SH est effectuée par action du mercapto-2 éthanol.

9. Procédé pour l'obtention d'une immunotoxine à durée d'action prolongée in vivo, caractérisé en ce qu'il consiste à coupler un anticorps ou d'un fragment d'anticorps avec une GPIR modifiée par le procédé selon l'une quelconque des revendications 1 à 8.

10. Procédé selon la revendication 9, caractérisé en ce que le couplage entre l'anticorps ou le fragment d'anticorps et la GPIR modifiée a lieu par l'intermédiaire d'un pont disulfure.

11. Procédé selon la revendication 10, caractérisé en ce que le couplage est effectué avec un réactif hétérobifonctionnel choisi parmi le N-succinimidyl (pyridyl-2 dithio)-3 propionate ou l'acide (pyridyl-2-disulfanyl)-3 propionique activé par l'éthyl-1 (diméthylamino-3 propyl)-3 carbodiimide.

**Claims for the contracting states: BE, CH, DE, FR, GB, IT, LI, LU, NL, SE**

1. Modified GPIR (ribosomes inactivating glycoproteins), having the ribosome-inhibiting activity of native GPIR and an in-vivo prolonged-action with respect to that of the GPIR, wherein said modified GPIR is obtained by treatment of a GPIR with periodate ions in the presence of primary amine in excess.

2. Modified GPIR according to claim 1, characterized in that at least one of the thiol groups of the GPIR is protected during the treatment with the periodate ions.

3. Modified GPIR according to one of claims 1 or 2, characterized in that the treatment with periodate ions is carried out in an aqueous solution of pH 5 to 7, at a temperature of 0 to 15°C, in the dark and for a period of 0.2 to 24 hours.

4. Modified GPIR according to one of claims 1 to 3, characterized in that the reaction solution contains between 1 and 10 mg/ml of reactive A chain, 10 to 50 mM of alkaline periodate and 50 to 500 mM of primary amine.

5. Modified GPIR according to one of claims 1 to 4, characterized in that the primary amine is an aliphatic amine selected from alkylamines, aminoacids, and hydrosoluble peptides.

6. Modified GPIR according to claim 5, characterized in that the aminoacid is selected from racemics or enantiomers of glycine, alanine, valine, leucine, isoleucine, arginine, aspartic acid, glutamic acid.

7. Process for the preparation of a modified GPIR according to one of claims 1 to 6, characterized in that an excess of alkaline periodate and a primary amine are reacted with a GPIR in aqueous solution.

8. Process according to claim 7, characterized in that the treatment with periodate ions is carried out in an aqueous solution of pH 5 to 7, at a temperature of 0 to 15°C, in the dark and for a period of 0.2 to 24 hours.

9. Process according to one of claims 7 or 8, characterized in that the reaction solution contains between 1 and 10 mg/ml of reactive GPIR, 10 to 50 mM of alkaline periodate and 50 to 500 mM of primary amine.

10. Process according to any one of claims 7 to 9, characterized in that before the oxidation reaction, the GPIR is treated with a conventional reagent protecting the groups SH, and in that said groups SH are released after the treatment by the $IO_4$ ions and the primary amine.

11. Process according to claim 10, characterized in that the reagent protecting the groups SH is selected from the 2,2′-dinitro-5,5′-dithio-dibenzoic acid and 3-(2-pyridyldisulfanyl)propionic acid, and in that the deprotection of the SH groups is achieved by the action of 2-mercaptoethanol.

12. Immunotoxin having a prolonged-action in vivo, resulting from the coupling of an antibody or antibody fragment with a modified GPIR according to any one of claims 1 to 6.

13. Immunotoxin according to claim 12, characterized in that the coupling between the antibody or antibody fragment and the modified GPIR is carried out by means of a disulfide bridge.

14. Immunotoxin according to claim 13, characterized in that the coupling is carried out with a heterobifunctional reagent selected from the N-succinimidyl-3-(2-pyridyldithio)propionate or the 3-(2-pyridyldisulfanyl)propionic acid activated by 1-ethyl-3-(3-dimethylaminopropyl) carbodiimide.

15. Anti-cancerous pharmaceutical composition containing as active principle an immunotoxin according to any one of claims 12 to 14.

**Claims for the contracting states: AT, ES, GR**

1. Process for the preparation of modified GPIR (ribosomes inactivating glycoproteins), having the ribosome-inhibiting activity of native GPIR and an in-vivo prolonged-action with respect to that of the GPIR, wherein said modified GPIR is obtained by treatment of a GPIR with periodate ions in the presence of a primary amine in excess.

2. Process according to claim 1, characterized in that at least one of the thiol groups of the GPIR is protected during the treatment with the periodate ions.

3. Process according to one of claims 1 or 2, characterized in that the treatment with periodate ions is carried out in an aqueous solution of pH 5 to 7, at a temperature 0 to 15°C, in the dark and for a period of 0.2 to 24 hours.

4. Process according to one of claims 1 to 3, characterized in that the reaction solution contains between 1 and 100 mg/ml of reactive A chain, 10 to 50 mM of alkaline periodate and 50 to 500 mM of primary amine.

5. Process according to one of claims 1 to 4, characterized in that the primary amine is an aliphatic amine selected from alkylamines, aminoacids, and hydrosoluble peptides.

6. Process according to claim 5, characterized in that the aminoacid is selected from racemics of enantiomers of glycine, alanine, valine, leucine, isoleucine, arginine, aspartic acid, glutamic acid.

7. Process according to any one of claims 1 to 6, characterized in that before the oxidation reaction, the GPIR is treated with a conventional reagent protecting the groups SH, and in that said groups SH are released after the treatment by the $IO_4$ ions and the primary amine.

8. Process according to claim 7, characterized in that the reagent protecting the groups SH is selected from the 2,2′-dinitro-5,5′-dithio-dibenzoic acid and 3-(2-pyridyldisulfanyl)propionic acid, and in that the deprotection of the SH groups is achieved by the action of 2-mercaptoethanol.

9. Process for the preparation of an immunotoxin having a prolonged-action in vivo, characterized in that it consists in coupling of an antibody or antibody fragment with a modified GPIR according to any one of claims 1 to 8.

10. Process according to claim 9, characterized in that the coupling between the antibody or antibody fragment and the modified GPIR is carried out by means of a disulfide bridge.

11. Process according to claim 10, characterized in that the coupling is carried out with a heterobifunctional reagent selected from the N-succinimidyl-3-(2-pyridyldithio)propionate or the 3-(2-pyridyldisulfanyl)propionic acid activated by 1-ethyl-3-(3-dimethylaminopropyl) carbodiimide.

**Patentansprüche für die Vertragsstaaten: BE, CH, DE, FR, GB, IT, LI, LU, NL, SE**

1. Modifiziertes GPIR (Ribosomen inaktivierendes Glykoprotein) mit einer die Ribosomen von nativem GPIR inhibierenden Aktivität und einer in bezug auf jene von GPIR verlängerte Wirkungsdauer in vivo, dadurch gekennzeichnet, daß es durch Behandlung eines GPIR mit Perjodationen in Gegenwart eines primären Amins im Überschuß erhalten ist.

2. Modifiziertes GPIR nach Anspruch 1, dadurch gekennzeichnet, daß mindestens eine der Thiolgruppen des GPIR während der Behandlung mit den Perjodationen geschützt ist.

3. Modifiziertes GPIR nach einem der Ansprüche 1 oder 2, dadurch gekennzeichnet, daß die Behandlung mit den Perjodationen in einer wässerigen Lösung mit pH 5 bis 7 bei einer Temperatur von 0 bis 15°C, in Dunkelheit und während einer Zeitdauer von 0,2 bis 24 Stunden stattfindet.

4. Modifiziertes GPIR nach einem der Ansprüche 1 bis 3, dadurch gekennzeichnet, daß die Reaktionslösung 1 bis 10 mg/ml reaktive Kette A, 10 bis 50 mM Alkaliperjodat und 50 bis 500 mM primäres Amin enthält.

5. Modifiziertes GPIR nach einem der Ansprüche 1 bis 4, dadurch gekennzeichnet, daß das primäre Amin ein aliphatisches Amin, ausgewählt aus Alkylaminen, Aminosäuren und wasserlöslichen Peptiden, ist.

6. Modifiziertes GPIR nach Anspruch 5, dadurch gekennzeichnet, daß die Aminosäure ausgewählt ist aus den Racematen oder den Enantiomeren von Glycin, Alanin, Valin, Leucin, Isoleucin, Arginin, Asparaginsäure, Glutaminsäure.

7. Verfahren zur Herstellung eines modifizierten GPIR nach einem der Ansprüche 1 bis 6, dadurch gekennzeichnet, daß man ein GPIR in wässeriger Lösung mit einem Überschuß an Alkaliperjodat und eines primären Amins reagieren läßt.

8. Verfahren nach Anspruch 7, dadurch gekennzeichnet, daß die Behandlung mit den Perjodationen in einer wässerigen Lösung mit pH 5 bis 7 bei einer Temperatur von 0 bis 15°C, in Dunkelheit und während einer Zeitdauer von 0,2 bis 24 Stunden stattfindet.

9. Verfahren nach einem der Ansprüche 7 oder 8, dadurch gekennzeichnet, daß die Reaktionslösung 1 bis 10 mg/ml reaktives GPIR, 10 bis 50 mM Alkaliperjodat und 50 bis 500 mM primäres Amin enthält.

10. Verfahren nach einem der Ansprüche 7 bis 9, dadurch gekennzeichnet, daß das GPIR weiter vor der Oxidationsreaktion mit einem klassischen Reagens zum Schutz der SH-Gruppen behandelt wird und daß die SH-Gruppen nach der Behandlung mit den $IO_4$-Ionen und dem primären Amin freigesetzt werden.

11. Verfahren nach Anspruch 10, dadurch gekennzeichnet, daß das Reagens zum Schutz der SH-Gruppen aus 2,2′-Dinitro-5,5′-dithio-dibenzoesäure und 3-(2-Pyridyldisulfanyl)propionsäure ausgewählt ist und daß die Entschützung der SH-Gruppen durch Einwirkung von 2-Mercaptoethanol erfolgt.

12. Immunotoxin mit verlängerter Wirkungsdauer in vivo, dadurch gekennzeichnet, daß es aus der Kopplung eines Antikörpers oder eines Antikörperfragments an ein modifiziertes GPIR nach einem der Ansprüche 1 bis 6 resultiert.

13. Immunotoxin nach Anspruch 12, dadurch gekennzeichnet, daß die Kopplung zwischen dem Antikörper oder Antikörperfragment und dem modifizierten GPIR unter Zwischenschaltung einer Disulfidbrücke erfolgt.

14. Immunotoxin nach Anspruch 13, dadurch gekennzeichnet, daß die Kopplung mit einem heterobifunktionellen Reagens, ausgewählt aus N-Succinimidyl-3-(2-Pyridyldithio)-propionat oder 3-(2-Pyridyl-disulfanyl)-propionsäure, aktiviert mit 1-Ethyl-3-(3-dimethlylaminopropyl)-carbodiimid, erfolgt.

15. Antikanzeröse pharmazeutische Zusammensetzung, dadurch gekennzeichnet, daß sie als Wirkstoff ein Immunotoxin nach einem der Ansprüche 12 bis 14 enthält.

**Patentansprüche für die Vertragsstaaten: AT, ES, GR**

1. Verfahren zur Herstellung eines modifizierten GPIR (Robosomen inaktivierenden Glykoproteins) mit einer die Ribosomen von nativem GPIR inhibierenden Aktivität und einer in bezug auf jene von GPIR verlängerte Wirkungsdauer in vivo, dadurch gekennzeichnet, daß es in der Behandlung eines GPIR mit Perjodationen in Gegenwart eines primären Amins im Überschuß besteht.

2. Verfahren nach Anspruch 1, dadurch gekennzeichnet, daß mindestens eine der Thiolgruppen des GPIR während der Behandlung mit den Perjodationen geschützt ist.

3. Verfahren nach einem der Ansprüche 1 oder 2, dadurch gekennzeichnet, daß die Behandlung mit den Perjodationen in einer wässerigen Lösung mit pH 5 bis 7 bei einer Temperatur von 0 bis 15°C, in Dunkelheit und während einer Zeitdauer von 0,2 bis 24 Stunden stattfindet.

4. Verfahren nach einem der Ansprüche 1 bis 3, dadurch gekennzeichnet, daß die Reaktionslösung 1 bis 10 mg/ml reaktive Kette A, 10 bis 50 mM Alkaliperjodat und 50 bis 500 mM primäres Amin enthält.

5. Verfahren nach einem der Ansprüche 1 bis 4, dadurch gekennzeichnet, daß das primäre Amin ein aliphatisches Amin, ausgewählt aus Alkylaminen, Aminosäuren und wässerlöslichen Peptiden, ist.

6. Verfahren nach Anspruch 5, dadurch gekennzeichnet, daß die Aminosäure ausgewählt ist aus den Racematen oder den Enantiomeren von Glycin, Alanin, Valin, Leucin, Isoleucin, Arginin, Asparaginsäure, Glutaminsäure.

7. Verfahren nach einem der Ansprüche 1 bis 6, dadurch gekennzeichnet, daß das GPIR weiter vor der Oxidationsreaktion mit einem klassischen Reagens zum Schutz der SH-Gruppen behandelt wird und daß die SH-Gruppen nach der Behandlung mit den $IO_4$-Ionen und dem primären Amin freigesetzt werden.

8. Verfahren nach Anspruch 7, dadurch gekennzeichnet, daß das Reagens zum Schutz der SH-Gruppen aus 2,2'-Dinitro-5,5'-dithio-dibenzoesäure und 3-(2-Pyridyldisulfanyl)propionsäure ausgewählt ist und daß die Entschützung der SH-Gruppen durch Einwirkung von 2-Mercaptoethanol erfolgt.

9. Verfahren zur Herstellung eines Immunotoxins mit verlängerter Wirkungsdauer in vivo, dadurch gekennzeichnet, daß es in der Kopplung eines Antikörpers oder eines Antikörperfragments mit einem durch das Verfahren nach einem der Ansprüche 1 bis 8 erhaltenen modifizierten GPIR besteht.

10. Verfahren nach Anspruch 9, dadurch gekennzeichnet, daß die Kopplung zwischen dem Antikörperfragment und dem modifizierten GPIR unter Zwischenschaltung einer Disulfidbrücke erfolgt.

11. Verfahren nach Anspruch 10, dadurch gekennzeichnet, daß die Kopplung mit einem heterobifunktionellen Reagens, ausgewählt aus N-Succinimidyl-3-(2-Pyridyldithio)-propionat oder 3-(2-Pyridyl-disulfanyl)-propionsäure, aktiviert mit 1-Ethyl-3-(3-dimethylaminopropyl)-carbodiimid, erfolgt.

Fig. 1

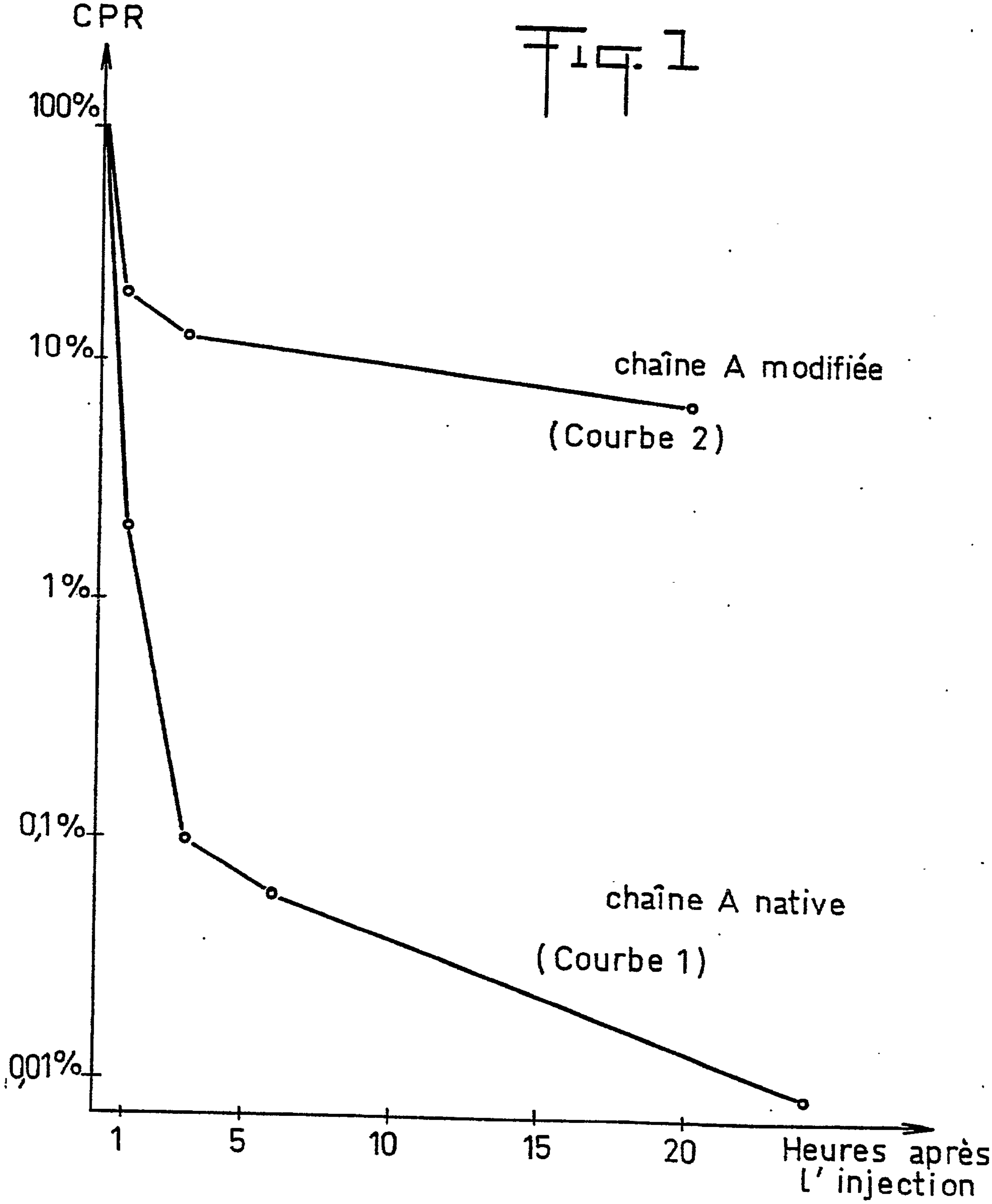
CPR
100%
10%
1%
0,1%
0,01%
chaîne A modifiée
(Courbe 2)
chaîne A native
(Courbe 1)
1
5
10
15
20
Heures après l'injection

Fig. 2

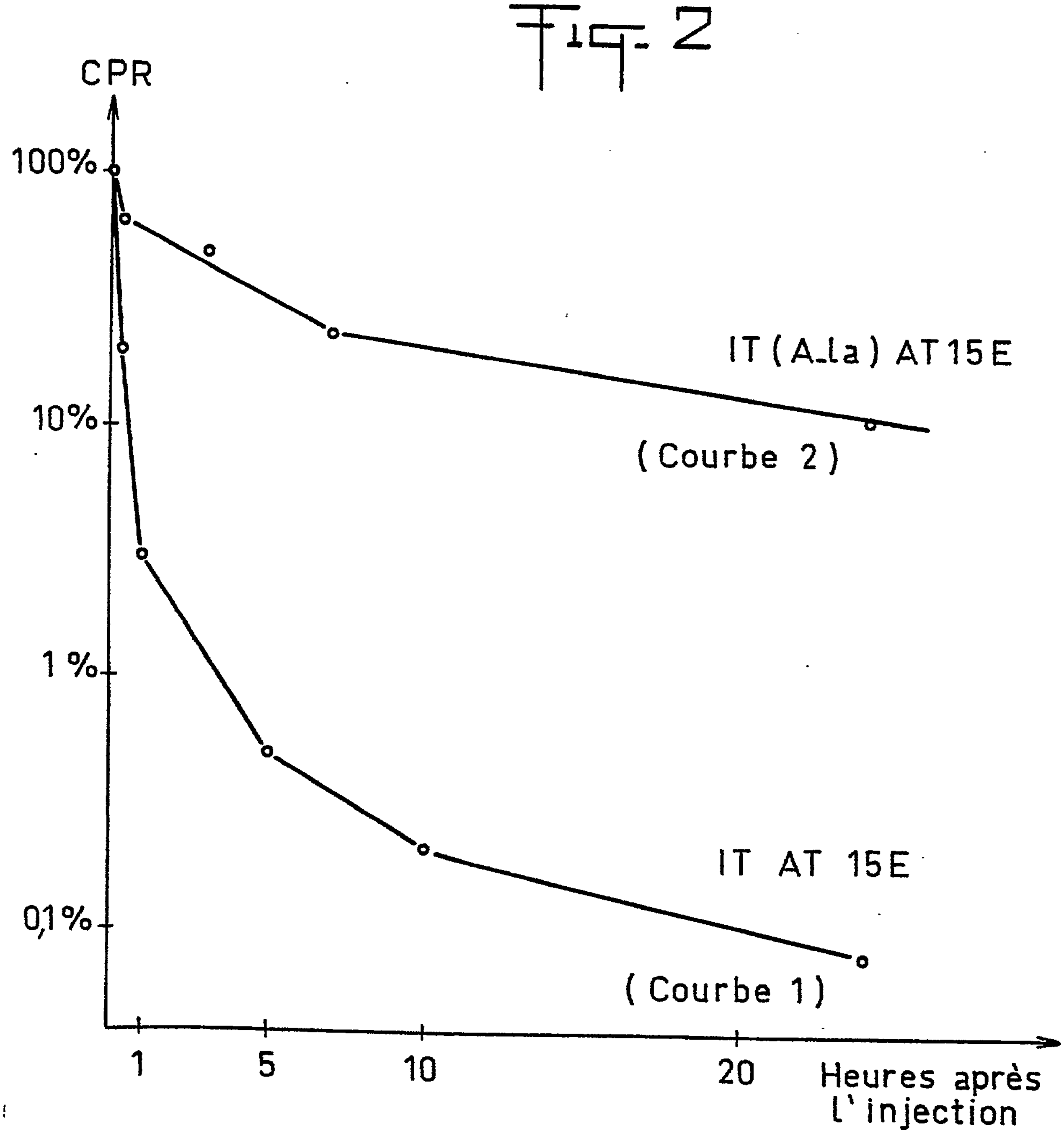
CPR
100%
10%
1 %
0,1%
IT (A-la) AT 15E
(Courbe 2)
IT AT 15E
(Courbe 1)
1
5
10
20
Heures après
l'injection